Europäisches Patentamt

(19) European Patent Office

Office européen des brevets

(11) Publication number: **0 068 740**
**B1**

(12) **EUROPEAN PATENT SPECIFICATION**

(45) Date of publication of patent specification: 22.03.89

(21) Application number: 82303155.4

(22) Date of filing: 17.06.82

(51) Int. Cl.⁴: **C 12 N 15/00,** C 07 H 21/04,
C 12 N 1/00

(54) Recombinant DNA cloning vectors and the eukaryotic and prokaryotic transformants thereof.

(30) Priority: 22.06.81 US 276445
26.03.82 US 362215

(43) Date of publication of application:
05.01.83 Bulletin 83/01

(45) Publication of the grant of the patent:
22.03.89 Bulletin 89/12

(84) Designated Contracting States:
BE CH DE FR GB IT LI LU NL SE

(56) References cited:
WO-A-82/03087

CHEMICAL ABSTRACTS, vol. 94, no. 13, 30th
March 19 1, page 169, no. 96942y, Columbus,
Ohio, US. J. DAVIES et al.: "A new selective
agent for eukaryotic cloning vectors" & AM. J.
TROP. MED. HYG. 1980, (5. SUPPL.), 1089-92
NATURE, vol. 287, 30th October 1980, pages
869-871, Basingstoke, GB. A. JIMENEZ et al.:
"Expression of a transposable antibiotic
resistance element in Saccharomyces"

(73) Proprietor: ELI LILLY AND COMPANY
307, East McCarty Street
Indianapolis Indiana 46285 (US)

(72) Inventor: Rao, Ramachandra Nagaraja
6818 Hoover Road
Indianapolis Indiana 46260 (US)
Inventor: Santerre, Robert Frank
619 Daffon Drive
Indianapolis Indiana 46227 (US)

(74) Representative: Hudson, Christopher Mark et al
Erl Wood Manor
Windlesham Surrey GU20 6PH (GB)

(56) References cited:
CHEMICAL ABSTRACTS, vol. 92, no. 11, 17th
March 1980, page 287, no. 90712k, Columbus,
Ohio, US. C. P. HOLLENBERG: "The expression
of bacterial antibiotic resistance genes in the
.yeast Saccharomyces cerevisiae" & DEV.
GENET. (AMSTERDAM) 1979, 1(PLASMIDS
MED., ENVIRON. COMMER. IMPORTANCE),
481-92

Courier Press, Leamington Spa, England.

(56) References cited:

Am. J. Trop. Med. Hyg. Vol. 29(5) 1980, pp. 1089-92

Antimicrobial Agents and Chemotherapy, Vol. 14, 1978, pp. 69-72

**Description**

The present invention concerns novel recombinant DNA cloning vectors that convey hygromycin B and G418 resistance to both eukaryotic and prokaryotic cells. The invention further concerns transformants of the aforementioned vectors.

The present invention is particularly important because it solves the problem of how to identify, manipulate, and stabilize cloned DNA sequences that lack a selectable function in eukaryotic and prokaryotic cells. Heretofore, the development and exploitation of recombinant DNA technology in eukaryotic cells has been retarded and made difficult because of 1) the general lack of suitable cloning vectors that contain markers that are selectable in eukaryotic cells; 2) the inability to quickly amplify desired DNA sequences in eukaryotic cells; and 3) the slow generation time of eukaryotic cells in culture.

In the prior art, Davies and O'Connor (Antimicrobial Agents and Chemotherapy *14*, 69—72 (1978)) disclose the selection and isolation of several ampramycin resistant strains. No plasmids possessing resistance to antibiotic G418 were isolated by these workers.

Jiminez and Davies, Nature, *287*, 869—871 (1980), and Davies and Jiminez, American Journal of Tropical Medicine and Hygiene *29* (5) Supp 1089—1091 (1980) disclose the introduction of an antibiotic G418 resistance gene into yeast which, however, confers phosphotransferase activity.

The present invention provides a recombinant DNA cloning vector that comprises a eukaryotic promoter, the plasmid pBR322 replicon, and

a) the ~7.5 kb *Bgl*II restriction fragment of plasmid pKC203 that conveys resistance to antibiotics hygromycin B and G418, or

b) the ~2.75 kb *Sal*I/*Bgl*II restriction fragment of plasmid pKC203 or plasmid pKC222 that conveys resistance to antibiotics hygromycin B and G418, or

c) the ~1.51 kb *Bgl*II/*Sac*I restriction fragment of plasmid pKC222 that conveys resistance to the antibiotic hygromycin B, or

d) the ~1.65 kb *Eco*RI/*Sal*I restriction fragment of plasmid pKC222 that conveys resistance to the antibiotic G418, or

e) a DNA sequence derived from the said ~7.5 kb restriction fragment that conveys resistance to either or both of antibiotics hygromycin B and G418;

the resistance to antibiotics hygromycin B and/or G418 being conveyed when the vector is transformed into a host cell that is sensitive to one or both of said antibiotics, the host cell being susceptible to transformation, cell division, and culture; subject to the limitations that the promoter is functional in mouse or yeast cells, that the antibiotic resistance conveying restriction fragments are adjacent to and, in a eukaryotic host cell, transcribed from the promoter, and that the DNA sequences conveying resistance to antibiotic G418 do not code for the enzyme phosphotransferase. Consequently, DNA sequences that are cloned onto the present versatile vectors can be conventionally manipulated and amplified in prokaryotic cells, thus avoiding the inconvenience and problems of doing the procedures in eukaryotic cells. Moreover, since the present vectors are both functional and selectable in eukaryotic cells, the vectors can be transformed following manipulation and amplification, directly into eukaryotic hosts without additional modification. This is not only advantageous but represents a significant advance in the technical art.

The invention also provides plasmid pKC222 which contains the ~2.75 kb *Sal*I/*Bgl*II restriction fragment of plasmid pKC203 as obtainable from *E. coli* JR225 ATCC 31912 ligated to the *Sal*I/*Bgl*II restriction fragment of plsamid pKC7, and which convers resistance to antibiotic ampicillin, hygromycin B and G418 when transformed into an *E. coli* cell.

The ability to clone DNA into eukaryotic host cells and to readily select the transformants is important for the further and more sophisticated development of recombinant DNA technology. Since transformation, and consequently acquisition of plasmid DNA, is a very low frequency event, such a functional test is a practical necessity for determining which cell(s), of among the millions of cells, has acquired the plasmid DNA. This is important because DNA sequences that are non-selectable can be inserted onto the vectors to cause insertional inactivation of a particular antibiotic resistance gene. Therefore, upon transformation, cells containing the vector and the particular DNA sequence of interest can be isolated by appropriate antibiotic selection. The present vectors are particularly useful because they are highly versatile and can be transformed and selected in any hygromycin B or G418 sensitive eukaryotic or prokaryotic cell that can divide and take up DNA. This is important because to date, most progress in the recombinant DNA art has involved the production of eukaryotic polypeptides such as, for example, proinsulin, insulin A chain, insulin B chain, human growth hormone, bovine growth hormone, interferon, somatostatin, thymosin α1, and the like, in prokaryotic hosts. Prokaryotic cells are incapable of correctly attaching sugar moieties to eukaryotic polypeptides. Therefore, the production of post translationally modified eukaryotic polypeptides, such as the glycosulated polypeptides, by recombinant DNA techniques is not possible without a eukaryotic host and appropriate eukaryotic recombinant DNA cloning vectors.

The present vectors fill this need and broaden the scope and application of recombinant DNA technology. Consequently, the commercial production of both unmodified and post-translationally modified proteins in eukaryotic cells is enhanced.

For purposes of the present invention as claimed herein, the following terms are as defined below.

3

Structural Gene—a DNA sequence that codes for a polypeptide.

Control Element—a DNA sequence that directs and regulates the transcription and expression of a structural gene.

Eukaryotic Promoter—a DNA sequence that in part promotes and regulates the expression of a structural gene in a eukaryotic cell.

Prokaryotic Replicon—a DNA sequence that controls and regulates the replication of DNA in a prokaryotic cell.

Recombinant DNA cloning Vector—any agent, including but not limited to plasmids, bacteriophages, and viruses, consisting of a DNA molecule to which one or more additional DNA segments can or have been added.

Transformation—the introduction of DNA into a recipient host cell that changes the genotype and consequently results in a stable and heritable change in the recipient cell.

Insertional isomer—one of the two or more possible recombinant DNA molecules formed when a DNA fragment is inserted at one of two or more compatible sites on the recipient DNA.

The present vectors are constructed by preparing a DNA sequence having one or two different structural genes and associated control sequence that convey resistance to either or both of antibiotics hygromycin B and G418 by a process which comprises treating plasmid pKC203 with

a) *Bg*/II restriction enzyme and isolating the 7.5 kb *Bg*/II restriction fragment that conveys resistance to both said antibiotics or

b) *Bg*/II and *Sa*/I restriction enzyme and isolating the 2.75 kb *Sa*/I/*Bg*/II restriction fragment that conveys resistance to both said antibiotics or

c) *Bg*/II and *Sac*I restriction enzyme and isolating the 1.51 kb *Sac*I/*Bg*/II restriction fragment that conveys resistance to antibiotic hygromycin B or

d) *Sa*/I and *Eco*RI restriction enzyme and isolating the 1.65 kb *Eco*RI/*Sa*/I restriction fragment that conveys resistance to antibiotic G418.

The DNA sequence thus obtained is ligated onto a second DNA sequence comprising a eukaryotic promoter and, if need be, a prokaryotic replicon. The DNA sequence obtained upon ligation is closed to form a plasmid which may be stabilized and maintained by transforming the plasmid into a host cell, culturing the transformed host in the presence of antibiotic and hygromycin B or antibiotic G418, and selecting the surviving cells.

The present invention, as exemplified herein, exploits bacterial plasmid DNA that convers resistance to the aminoglycoside antibiotics hygromycin B and G418, for constructing novel plasmids that contain and express the resistance in hygromycin B or G418 sensitive eukaryotic and prokaryotic host cells. Thus, the present invention is useful for cloning DNA into virtually any type of cell. Furthermore, the ability of the vectors exemplified herein to confer resistance to antibiotics that are toxic in both eukaryotic and prokaryotic cells also provides a functional test for selecting and stabilizing cells that have acquired the vectors.

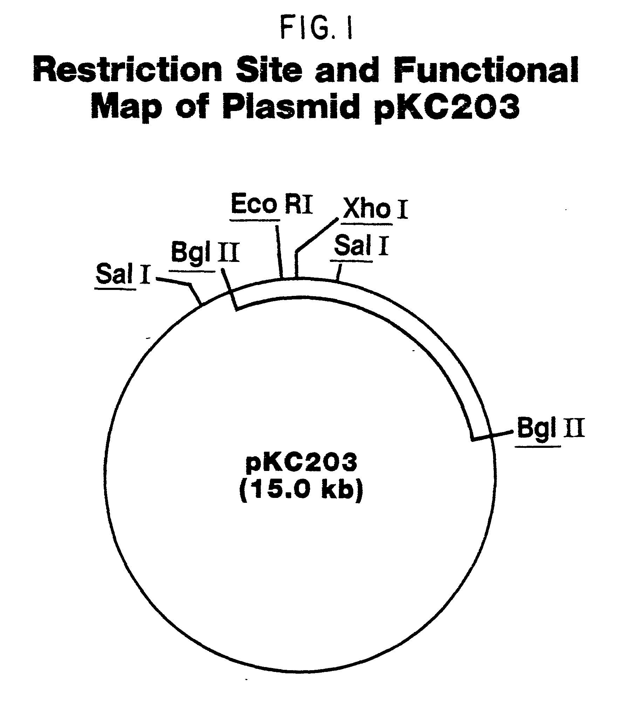

The particular bacterial plasmid DNA sequence used in the present illustrative constructions, is the 7.5 kb *Bg*/II restriction fragment, or derivatives thereof, of plasmid pKC203. Plasmid pKC203 is approximately 15 kb in size and can be isolated readily from *E. coli* JR225 by conventional procedures. Strain *E. coli* JR225 is both known in the art (Davis and O'Connor, 1978, Antimicrobial Agents and Chemotherapy 14(1):69), and also deposited and made part of the stock culture collection of the American Type Culture Collection, Rockville, Maryland, from which it is available to the public without restriction under the number ATCC 31912. A restriction and functional map of plasmid pKC203 is presented in Figure 1 of the accompanying drawings. Figure 1 and all subsequent figures are not drawn to scale.

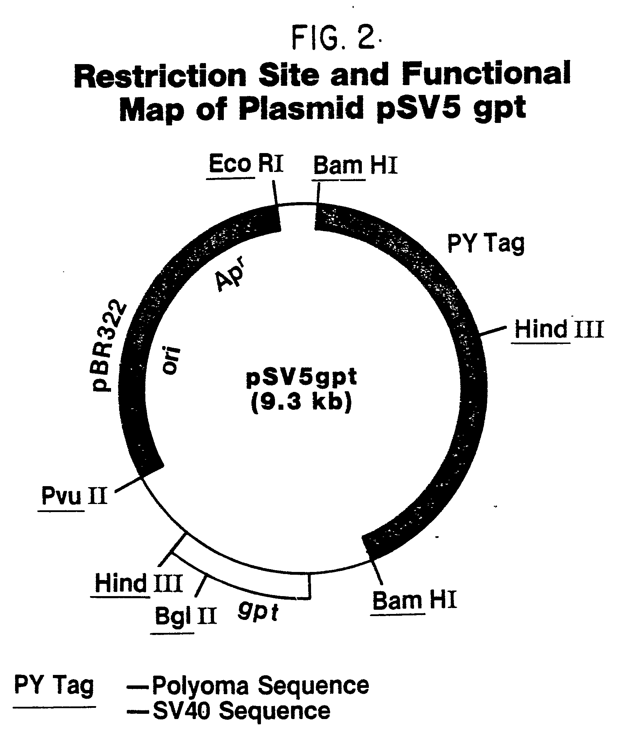

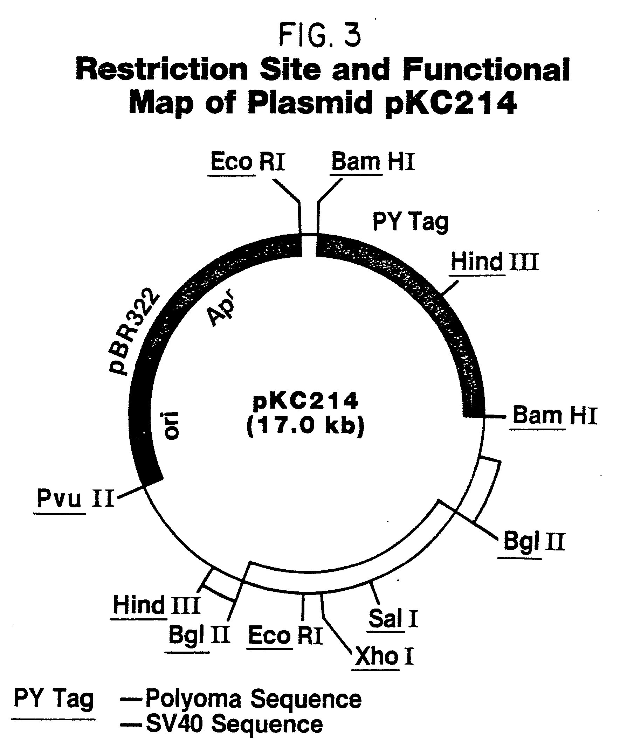

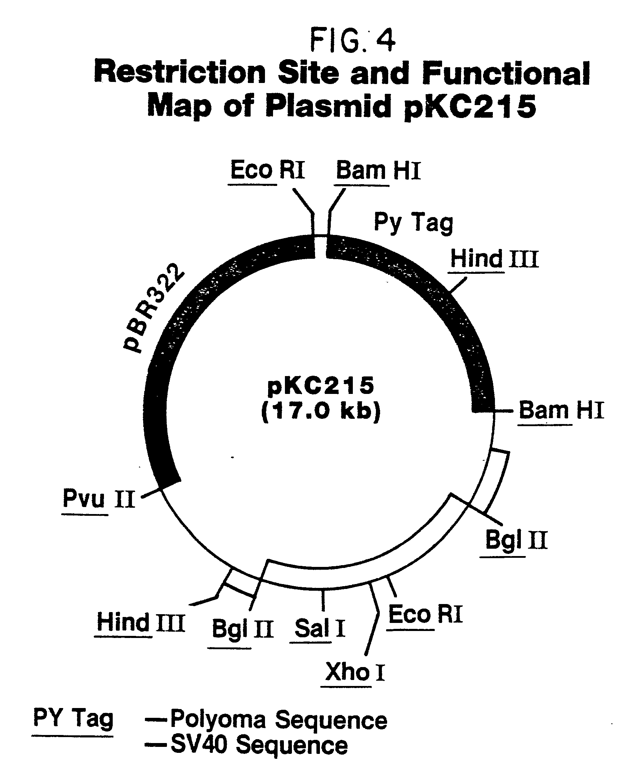

The 7.5 kb *Bg*/II restriction fragment of pKC203 comprises the structural genes and control elements for expression of resistance to antibiotics hygromycin B and G418. This fragment was ligated onto plasmid pSV5 gpt, a known plasmid whose construction is described in Mulligan and Berg, 1980, Science 209 (4463):1422. Plasmid pSV5 gpt is approximately 9.3 kb and contains both the origin of replication and the ampicillin resistance marker of plasmid pBR322 and also a functional SV40 early promoter which controls the transcription of a gene coding for xanthine-guanine phosphoribosyltransferase (gpt). A restriction site and functional map of plasmid pSV5 gpt is presented in Figure 2 of the accompanying drawings. As will be apparent to those skilled in the art, plasmid pSV5 gpt can replicate both in *E. coli* and also in eukaryotic cells such as, for example, mammalian cells. Moreover, the unique *Bg*/II restriction site in plasmid pSV5 gpt allows for the direct ligation with the 7.5 kb *Bg*/II restriction fragment of plasmid pKC203. Thus, the hygromycin B and G418 resistance conferring 7.5 kb fragment can be ligated onto *Bg*/II treated plasmid pSV5 gpt. Since two possible orientations of the 7.5 kb fragment are equally probable, the ligation of the 7.5 kb fragment onto plasmid pSV5 gpt results in plasmids of two types, designated herein as plasmids pKC214 and pKC215. Restriction and functional maps of plasmids pKC214 and pKC215 are presented respectively in Figures 3 and 4 of the accompanying drawings.

In the illustrative plasmids pKC214 and pKC215, the eukaryotic promoter is adjacent to the genes, including the associated control elements, for hygromycin B and G418 resistance and is exemplified by the SV40 early promoter. The eukaryotic promoter controls the transcription and in part regulates the expression of the resistance genes when transformed into eukaryotic host cells. In addition, the present

4

plasmids undergo chromosomal integration in eukaryotic hosts and therefore replicate along with and as part of the chromosomes during normal eukaryotic cell division.

Although the eukaryotic promoter herein exemplified in the illustrative plasmids pKC214 and pKC215 is the SV40 early promoter (O'Hare et al., 1981, Proc. Nat. Acad. Sci. USA 78(3) 1527, and Mulligan and Berg, 1980, Sci 209:1422) many other eukaryotic promoters can also be used. Other illustrative eukaryotic promoters include, but are not limited to, the SV40 late promoter (Hamer and Leder, 1979, Nature 281:35), HSVITK promoter (Pellicer et al., 1978, Cell 14:133), adenovirus promoter (Thummel and Tjian, 1981, Cell 23:825), adenovirus 2 (Ad 2) late promoter (Solnick, 1981, Cell 24:135), polyoma promoter (Colantuoni et al., 1980, Proc. Nat. Acad. Sci. USA 77(7):3850), mouse sarcoma virus promoter (VanBeveren et al., 1981, Nature 289:258), yeast trp-1 promoter (Stinchcomb et al., 1979, Nature 282:39), yeast leu 2 promoter (Ratzkin and Carbon, 1977, Proc. Nat. Acad. Sci. USA 74(2):487, yeast his 3 promoter (Broach et al., 1979, Gene 8:121), the yeast alcohol dehydrogenase promoter, and the yeast cytochrome c promoter (Guarente and Ptashne, 1981, Proc. Nat. Acad. Sci. USA 78(4):2199).

While the construction of the illustrative plasmids pKC214 and pKC215 involved a BgIII insertion into the pSV5 gpt gene downstream from the early SV40 promoter, other analogous constructions involving one or more of the above listed eukaryotic promoters can be made. For example, a BgIII linear DNA fragment containing the hygromycin B and G418 resistance genes can be obtained from plasmid pKC203, or a derivative thereof, by treating the plasmid with BgIII restriction enzyme according to conventional procedures. This fragment can be used directly or synthetic DNA linkers, known in the art, can be ligated to the resistance conferring fragment and the thus prepared fragment can be cloned downstream to an appropriate eukaryotic promoter such as, for example, the Ad 2 or the yeast cytochrome c promoter. Upon ligation with a prokaryotic replicon, these constructions, exemplified herein by plasmids pGD1, pGD2, pGD3, and pGD4, are functional in eukaryotic and prokaryotic host cells and are thus within the scope of the present invention. Moreover, it is understood and will be apparent to those skilled in the art that additional constructions involving different eukaryotic promoters can be made and that some eukaryotic promoters and constructions are preferred over others for use in certain hosts.

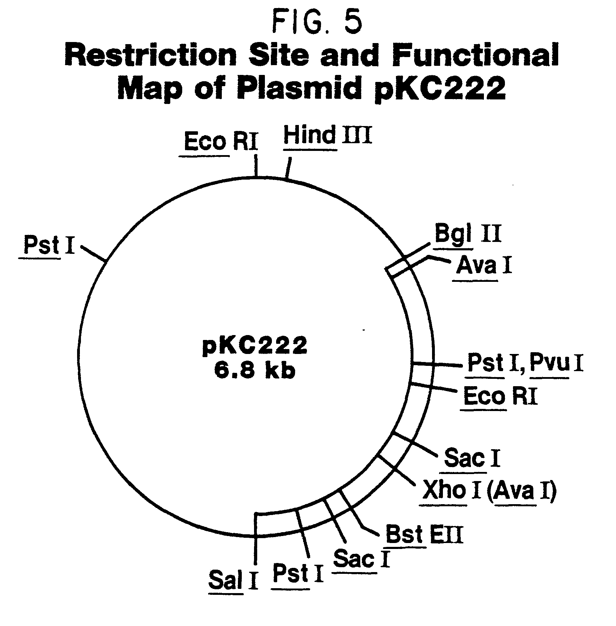

The particular hygromycin B and G418 resistance genes and control elements, exemplified in illustrative plasmids pKC214, pKC215, pGD1, pGD2, pGD3 and pGD4, comprise the 7.5 kb BgIII fragment of plasmid pKC203. Moreover, within this BgIII fragment, the DNA sequence that codes for resistance has been further localized to the 2.75 kb SaII/BgIII fragment. A restriction and functional map of the 2.75 kb SaII/BgIII fragment is presented as part of plasmid pKC222 in Figure 5 of the accompanying drawings. Plasmid pKC222 is useful for isolating the individual genes and control elements that confer resistance to particular antibiotics. For example, the 1.51 kb BgIII/SacI fragment of plasmid pKC222 comprises the hygromycin B resistance gene and control element while the 1.65 kb EcoRI/SaII fragment comprises the gene and control element that confers resistance to G418. Furthermore, it is believed that the gene and associated control-element that confers resistance to G418 also simultaneously confers resistance to antibiotics apramycin and tobramycin in sensitive hosts such as, for example, E. coli. Therefore, recombinant DNA cloning vectors that confer resistance to one, more than one, or all of the antibiotics can be constructed by cloning an appropriate plasmid pKC203 or pKC222 DNA fragment downstream to an appropriate eukaryotic promoter. Upon being provided with a prokaryotic replicon, the vectors, exemplified herein by plasmids pGD10, pGD11, pGD12, pGD13, pGD14, and pGD15, are functional in both eukaryotic and prokaryotic host cells and are thus within the scope of the present invention. It will be understood that the present invention is in no way limited by the above postulated additional resistance conferring activity of the G418 resistance gene.

The 7.5 kb BgIII fragment of plasmid pKC203 also contains a prokaryotic replicon and therefore can be self ligated to form a plasmid. The resulting plasmid, designated as pSC701, is functional in E. coli and is useful for generating derivative plasmids which are useful as starting materials. Thus, the plasmid pSC701 DNA can be HaeII digested and then ligated to produce plasmids pKC257 and pKC259. Plasmid pKC257 is ~4.2 kb and confers resistance to hygromycin B while plasmid pKC259 is ~5.0 kb and confers resistance to both hygromycin B and apramycin. Sau3AI digestion of plasmid pKC257 DNA followed by ligation results in a still smaller plasmid, designated as pKC261. This plasmid also confers resistance to hygromycin B.

The above derivative plasmids are functional in E. coli and are useful as starting material for constructing vectors of the present invention. Thus, insertion of plasmid pKC259 into pSV5 gpt results in plasmids pLO314 and pLO315; insertion of plasmid pKC257 into pSV5 gpt results in plasmids pLO316 and pLO317; and insertion of plasmid pKC261 into pSV5 gpt results in plasmids pLO318 and pLO319. All of the aforementioned pLO plasmids are functional in both prokaryotic and eukaryotic host cells and thus are within the scope of the present invention.

Additional plasmid starting materials can also be constructed. For example, insertion of the plac-containing HaeII restriction fragment of plasmid pUR222, (the construction of which is disclosed in Rüther, 1981, Nucleic Acids Research 9:4087), into plasmid pKC261 results in plasmid pKC275. The latter plasmid is ~3.6 kb, confers resistance to hygromycin B, and can be inserted into plasmid pSV5 gpt to form illustrative plasmids pLO320 and pLO321. Furthermore, insertion of a 2 μ DNA-containing restriction fragment of plasmid YEp24 into pKC259 results in plasmid pKC264, insertion of the ~2.5 kb BgIII/SaII fragment of pKC259 into appropriately cut YEp24 results in plasmid pKC273, and insertion of the ~3.2 kb PstI fragment of plasmid pKC264 into pBR322 results in plasmid pLO378. Both plasmids pLO378 and pKC273

are functional in yeast and *E. coli* and consequently are within the scope of the present invention. Construction of the aforementioned and other vectors using the foregoing plasmid starting materials is further and more specifically disclosed in Examples 25 to 42 below.

It will be understood that those of ordinary skill can construct or isolate still other DNA sequences that also confer resistance to hygromycin B and G418, either individually or in combination, and that these sequences can be used in place of the resistance genes and control elements exemplified herein. Moreover, functional derivatives of the 7.5 kb *Bg*/II fragment or the 2.75 kb *Sal*I/*Bg*/II subfragment can be constructed by adding, eliminating, or substituting certain nucleotides in accordance with the genetic code. Those of ordinary skill will understand that use of such derivatives and also other hygromycin B and G418-conferring DNA segments results in vectors that are within the scope of the present invention.

Although the prokaryotic replicon exemplified herein in the illustrative plasmids is the plasmid pBR322 replicon, many other replicons can also be used for making similar constructions. Other illustrative prokaryotic replicons include, but are not limited to, the pMB1 replicon (Betlach *et al.,* 1976, Fed. Proc. 35:2037), NR1 replicon (Rownd and Mickel, 1971 Nature 234:40), RK2 replicon (Beringer, 1974, J. Gen. Microbiol. 84:188), RK6 replicon (Kontomichalou *et al.,* 1970, J. Bacteriol. 104:34), pSC101 replicon (Cohen and Chang, 1977, J. Bacteriol. 132:734), RP1 replicon (Grinsted *et al.,* 1972, J. Bacteriol. 110:529), RP4 replicon (1977, Nagahari *et al.,* Gene 1:141), RSF1010 replicon (Guerry *et al.,* 1974, J. Bacteriol. 117:619), PUB110 replicon (Gryczan, *et al.,* 1978, J. Bacteriol. 134:318), and the SLP1.2 replicon (Bibb *et al.,* Nature 284:526. It is understood and will be apparent to those skilled in the art that many additional prokaryotic replicons can be constructed and that generally some prokaryotic replicons are preferred and will be selected over others for use in certain hosts. For example, the RSF1010, PUB110, and the SLP1.2 replicons are respectively preferred for use in *Pseudomonas, Bacillus* and *Streptomyces,* while the other replicons cited above are preferred for use in *E. coli.*

The vectors of the present invention are highly versatile and are functional in almost any prokaryotic or eukaryotic host cell. The only requirements are 1) that the host cell be capable of division and culture; 2) that the host cell be competent for transformation; and 3) that the non-transformed host cell be sensitive to and thus killed by either or both hygromycin B and G418. Therefore, the present vectors are useful in bacteria, fungi, yeast, plant cells, animal cells, and free living unicellular eukaryotes.

The wealth of genetic and biochemical information about *E. coli* K12 makes it a convenient and preferred prokaryotic host cell for purposes of the present invention. Other preferred prokaryotic host cells are bacteria, including but not limited to *E. coli, E. coli* K12 BE827. Preferred eukaryotic host cells are fungi and yeast; cells susceptible to culture that are derived from tissue of multicellular organisms, including but not limited to a mammalian cell, murine mammalian cell, mouse cell, Mouse Ltk⁻ cell.

As described above, the vectors of the present invention are functional in virtually any type of prokaryotic or eukaryotic host cell and confer the desired antibiotic resistance to hygromycin B and G418 sensitive cells such as, for example, *E. coli* K12 BE827, *E. coli* K12 BE783, *E. coli* K12 BE1041, Mouse Ltk⁻ cells, and *Saccharomyces cerevisiae.* Thus, the transformants of the present invention, including but not limited to illustrative transformants *E. coli* K12 BE827/pKC214, *E. coli* K12 BE827/pKC215, Mouse Ltk⁻/pKC214, Mouse Ltk⁻/pKC215, *E. coli* K12 BE783/pKC273, and *Saccharomyces cerevisiae*/pKC273, express resistance to either or both of the aforementioned antibiotics and are therefore useful, under appropriate selection conditions, for propagating and maintaining the present vectors. Those skilled in the art will further recognize that non-selectable structural genes can be cloned into the vectors and that, following transformation and subsequent culturing under hygromycin B or G418 selection pressure in mammalian or other host cells, the cloned genes can be stabilized, maintained, and expressed. Only host cells thus transformed can survive in the presence of hygromycin B or G418 so therefore, the vectors of the present invention allow for the easy isolation and initial identification of transformants.

The large number of host cells that can be transformed with the vectors of the present invention is important because it allows for the easy amplification and manipulation of eukaryotic vectors in prokaryotic host cells. This is particularly advantageous because the genetic background of prokaryotes, such as *E. coli* and the like, is well known and conventional recombinant DNA procedures are applicable and can be conveniently done in such systems. Consequently, the present recombinant DNA cloning vectors, including those that contain selectable or non-selectable structural genes, can be first manipulated and amplified in prokaryotic cells and then transformed into eukaryotic host cells for expression, thus avoiding the serious problems associated with being restricted exclusively to eukaryotic systems.

While all the embodiments of the present invention are useful, some of the present recombinant DNA cloning vectors and transformants are preferred. Accordingly, preferred vectors are plasmids pKC214, pKC215, and pKC273; preferred prokaryotic transformants are *E. coli* K12 BE827/pKC214, *E. coli* K12 BE827/pKC215, and *E. coli* K12 BE783/pKC273; and preferred eukaryotic transformants are Mouse Ltk⁻/pKC214, Mouse, Ltk⁻/pKC215, and *Saccharomyces cerevisiae*/pKC273.

The utility of the vectors and transformants of the present invention is broad and allows for the greater and more rapid application of recombinant DNA technology to eukaryotic systems. For example, the ability of the present vectors, including plasmids, bacteriophages, and viruses, to confer resistance to antibiotics that are toxic to both eukaryotic and prokaryotic cells provides a functional test for selecting transformants. This is important because such a test is a practical necessity for determining and selecting the particular cells that have acquired vector DNA. Additional DNA sequences, that lack functional tests for their presence, can be inserted onto the present vectors and then transformants containing the non-selectable DNA can be isolated by hygromycin B or G418 selection. Such non-selectable DNA sequences include, but are not limited to, genes that specify a post-translationally modified protein such as, for example, fibronectin or hepatitis B antigen, both of which are post-translationally glycosylated by eukaryotic cells.

More particularly a non-selectable gene sequence can be inserted, for example, into the *Pvul* site of plasmids, such as, for example, illustrative plasmids pGD14 and pGD15, that contain the 2.75 kb *Sall/Bglll* restriction fragment of plasmid pKC203. Such an insertion inactivates the hygromycin B resistance gene and thus allows for the easy identification of transformants containing the recombinant plasmid. This is done by first selecting for G418 resistance and, secondarily, identifying those G418 resistant transformants that are not resistant to hygromycin B. In a similar manner, insertion of a gene sequence of interest at, for example, the *Xhol* site inactivates the G418 resistance gene. Thus transformants carrying this recombinant plasmid also can be identified easily by first selecting for hygromycin B resistance and, secondarily, identifying those hygromycin B transformants that are not resistant to G418. Therefore, the ability to select for antibiotic resistance in eukaryotic or prokaryotic transformants allows for the efficient isolation of the extremely rare cells that contain the particular non-selectable DNA of interest.

The functional test for antibiotic resistance, as described herein above, can also be used to identify DNA sequences that act as control elements and direct expression of an individual antibiotic resistance gene. Such sequences, including but not limited to promoters, attenuators, repressors, inducers, ribosomal binding sites, and the like, can be used to control the expression of other structural genes in both eukaryotic and prokaryotic cells.

The hygromycin B and G418 resistance-conferring vectors of the present invention are also useful for stabilizing linked DNA sequences of various sorts. These genes or fragments, covalently linked to the hygromycin B or G418 resistance gene and propagated either in eukaryotes or prokaryotes, can be maintained by exposing the transformants to levels of hygromycin B or G418 that are toxic to non-transformed cells. Therefore, transformants that lose the vector, and consequently any covalently linked DNA, die and are eliminated from the culture. Thus, the vectors of the present invention can stabilize and maintain any DNA sequence of interest.

The cloning vectors and transformants of the present invention not only provide for the commercially feasible production of both unmodified and post-translationally modified polypeptides in eukaryotic cells, but also for improved yields of various products that are currently produced in prokaryotic and eukaryotic cells.

The capability for inserting and stabilizing such DNA sequences in eukaryotic and prokaryotic cells is important because the production, using recombinant DNA methodology, of certain antibiotics and post-translationally modified proteins requires a eukaryotic host. Moreover, the ability of the present vectors to be functional in prokaryotic cells such as, for example, *E. coli*, allows for their easy manipulation and amplification, thus circumventing the problems associated with eukaryotic cells in which such procedures are difficult if not impossible to do.

The following examples further illustrate and detail the invention disclosed herein. Both an explanation of and the actual procedures for constructing the invention are described where appropriate.

Example 1
Plasmid pKC203
A. Isolation of plasmid DNA from *E. coli* JR225
The bacterium *E. coli* JR225 (ATCC No. 31912) was cultured in TY broth (1% tryptone, 0.5% yeast extract, 0.5% sodium chloride, pH 7.4) with 100 µg/ml of antibiotic hygromycin B according to conventional microbiological procedures. After 18 hours incubation, about 0.5 ml of the culture was transferred to a 1.5 ml Eppendorf tube and centrifuged for about 15 seconds. Unless otherwise indicated, all the manipulations were done at ambient temperature. The resultant supernatant was carefully removed with a fine-tip aspirator and the cell pellet was suspended in about 100 µl of freshly prepared lysozyme solution which contained 2 mg/ml lysozyme, 50 mM glucose, 10 mM CDTA (cyclohexane diaminetetraacetate) and 25 mM Tris-HCl (pH 8.0). After incubation at 0°C for 30 minutes about 200 µl of alkaline SDS (sodium dodecyl sulfate) solution (0.2 N NaOH, 1% SDS) was added and the tube was gently vortexed and then maintained at 0°C for 15 minutes. Next about 150 µl of 3 M sodium acetate (prepared by dissolving 3 moles of sodium acetate in a minimum of water, adjusting the pH to 4.8 with glacial acetic acid, and then adjusting the volume to 1 l) was added and the contents of the tube were then mixed gently by inversion for a few seconds during which time a DNA clot formed.

The tube was maintained at 0°C for 60 minutes and then centrifuged for 5 minutes to yield an almost clear supernatant. About .4 ml of the supernatant was transferred to a second centrifuge tube to which 1 ml of cold ethanol was added. After the tube was held at −20°C for 30 minutes, the resultant precipitate was collected by centrifugation (2 minutes) and the supernatant was removed by aspiration. The thus collected pellet was dissolved in 100 µl of 0.1 M sodium acetate/0.05 M Tris-HCl (pH 8) and was reprecipitated by the addition of 2 volumes of cold ethanol. After 10 minutes at 20°C, the precipitate was collected by centrifugation, as described above, and constitutes the desired *E. coli* JR225 plasmid DNA.

B. Transformation of *E. coli* JR225 plasmid DNA and isolation of plasmid pKC203

The *E. coli* JR225 plasmid DNA pellet was dissolved in about 100 µl of 0.1 M sodium acetate/0.05 M Tris-HCl (pH 8) and precipitated with 2 volumes of cold ethanol. The resultant plasmid DNA, was collected and then dissolved in about 40 µl of water or dilute buffer, and finally used to transform *E. coli* K12 BE827 in substantial accordance with the transformation method of Wensink, 1974, Cell 3:315. *E. coli* K12 BE827 has been deposited and made part of the stock culture collection of the American Type Culture Collection, Rockville, Maryland, from which it is available to the public without restriction under the number ATCC 31911. The resultant transformants were selected on TY agar (1% tryptone, 0.5% yeast extract, 0.5% sodium chloride, 1.5% agar, pH 7.4) containing 200 µg/ml of antibiotic hygromycin B. Some of the transformants, as shown by gel electrophoresis (Rao and Rogers, 1978) and other tests, contained both large and smaller (15 kb) plasmids and were resistant to both antibiotics ampicillin and hygromycin B. Other transformants contained only the smaller 15 kb plasmid and were resistant to antibiotics hygromycin B and G418 but were sensitive to ampicillin.

Transformants of the latter type were plated on TY agar containing 1 mg/ml of antibiotic hygromycin B and were cultured using standard microbiological techniques. The resultant cells were used, according to the procedure of Example 1A, to isolate the above described 15 kb hygromycin B and G418 resistance conferring plasmid, hereinafter designated as plasmid pKC203. The presence of the antibiotic hygromycin B and G418 resistance genes on plasmid pKC203 was confirmed by subsequent transformation and selection analysis.

Example 2

Isolation of the antibiotic hygromycin B and G418 resistance genes and control elements

About 5 µg of plasmid pKC203 DNA were treated with *Bg*/ll restriction enzyme according to the instructions and under the conditions specified by the manufacturer*. Of the 7.5 kb, 5.8 kb, and 0.5 kb fragments recovered, the 7.5 kb *Bg*/ll fragment contained the desired antibiotic hygromycin B and G418 resistance genes and control elements. This was confirmed by subsequent transformation and selection analysis which showed that cells that are normally sensitive to antibiotics hygromycin B and G418 are resistant to the antibiotics upon transformation with the 7.5 kb *Bg*/ll fragment.

*Restriction enzymes and instructions can be readily obtained from the following sources:

Bethesda Research Laboratories Inc.
Box 6010
Rockville, Maryland 20850

Boehringer Mannheim Biochemicals
7941 Castleway Drive
P.O. Box 50816
Indianapolis, Indiana 46250

New England Bio Labs., Inc.
283 Cabot
Beverly, Massachusetts 01915

Research Products
Miles Laboratories Inc.
Elkhart, Indiana 46515

Example 3

Construction of plasmids pKC214 and pKC215 and transformants *E. coli* K12 BE827 pKC214 and *E. coli* K12 BE827/pKC215

Plasmid pSV5 gpt, the construction of which is described in Mulligan and Berg, 1980, Science 209(4463):1422, has a unique *Bg*/ll site within the gpt gene. Cloning as described below, allows for the expression of the antibiotic hygromycin B and G418 resistance genes.

About 5 µg of plasmid pSV5 gpt DNA were treated with *Bg*/ll restriction enzyme according to the instructions and under the conditions specified by the manufacturer. After the enzyme was inactivated by heating at 70°C for 5 minutes, about 1 µg of the DNA was mixed in a 1:1 ratio with the 7.5 kb *Bg*/ll fragment of pKC203. The fragments were joined using T4 DNA ligase according to the instructions and under the conditions specified by the manufacturer*.

*T4 DNA ligase and instructions can be readily obtained from the following source:

Bethesda Research Laboratories
Box 6010
Rockville, Maryland 20850

The ligated mixture was used to transform *E. coli* K12 BE827 in substantial accordance with the transformation method of Wensink, 1974, Cell 3:315, on TY plates containing 100 µg/ml each of antibiotics ampicillin and apramycin. The recombinant clones were plated on TY plates containing 100 µg/ml of ampicillin and 200 µg/ml of antibiotic hygromycin B. About half of the antibiotic hygromycin B resistant recombinant clones contained plasmid pKC214 (Figure 3) while the remainder contained plasmid pKC215 (Figure 4).

Plasmid DNA from various of the above clones was isolated, according to the procedure of Example 1A, and conventionally distinguished by restriction enzyme analysis. In this way, the constructed plasmids pKC214 and pKC215 and the constructed transformants E. coli K12 BE827 pKC214 and E. coli K12 BE827/pKC215 were identified and subsequently isolated for future use.

Example 4
Construction of Mouse Ltk⁻/pKC214

Mouse Ltk⁻ cells were conventionally cultured for regular cell maintenance in a medium comprising minimum essential medium with Earle's salts and non-essential amino acids (Eagle, 1959, Science 130:432), 10% v/v newborn calf serum, and 292 µg/ml glutamine. The thus cultured Mouse Ltk⁻ cells were then transformed with plasmid pKC214 in substantial accordance with the protocol described in Wigler et al. 1979, Proc. Nat. Acad. Sci. USA 76(3):1373, except that 1) 100—300 ng of plasmid DNA was added to each plate in 1 ml of calcium phosphate precipitate; and 2) the culture medium was as described above. After 4 hours incubation at 37°C, the medium was replaced with fresh medium and the cells were allowed to incubate for an additional 20 hours. At that time antibiotic hygromycin B selection pressure was applied. This was done by changing the medium to a selection medium which contained the above described medium and also about 75—1000 µg/ml of antibiotic hygromycin B. The concentration of the antibiotic that is preferred for the selecting medium is 200 µg/ml. The selecting medium was changed after the first day, then two days after that, and finally after every third day over the 2—3 weeks in which transformant clones arose. Colonies were harvested by hand using a pipette and were grown into mass culture under continued selection pressure. The thus cultured antibiotic hygromycin B resistant cells constitute the desired Mouse Ltk⁻/pKC214 transformants.

Example 5
Construction of Mouse Ltk⁻/pKC215

Mouse Ltk⁻/pKC215 cells were constructed in substantial accordance with the teaching of Example 4, except that plasmid pKC215, rather than plasmid pKC214, was used in the transformation procedure. The thus constructed Mouse Ltk⁻/pKC215 transformants were then grown into mass culture.

Example 6
Resistance of transformants to antibiotics hygromycin B and G418

The ability of plasmids pKC203, pKC214, and pKC215 to confer resistance to antibiotics hygromycin B and G418 were determined by testing transformed and non-transformed E. coli and Mouse Ltk⁻ cells for growth on media with varying amounts of the antibiotics. The media and culture conditions for E. coli and Mouse Ltk⁻ cells are substantially as described respectively in Examples 1A and 4. The results of the test are presented below in Tables 1 and 2 wherein '+' indicates growth, '−' indicates no growth, and 'N/T' indicates not tested.

TABLE 1
Resistance to antibiotic hygromycin B

| | Antibiotic concentration of culture medium | | |
| --- | --- | --- | --- |
| Cell type | 0 µg/ml | 200 µg/ml | 400 µg/ml* |
| E. coli K12 | + | − | − |
| E. coli K12 BE827/pKC203 | + | + | + |
| E. coli K12 BE827 | + | − | − |
| E. coli K12 BE827/pKC214 | N/T | + | N/T |
| E. coli K12 BE827/pKC215 | N/T | + | N/T |
| Mouse Ltk⁻ | + | − | N/T |
| Mouse Ltk⁻/pKC214 | + | + | + |
| Mouse Ltk⁻/pKC215 | + | + | + |

*Mouse Ltk⁻/pKC214 and Mouse Ltk⁻/pKC215 cells not only replicated at 400 µg/ml but also survived higher concentrations exceeding 1000 µg/ml.

TABLE 2
Resistance to antibiotic G418

| Cell type | Antibiotic concentration of culture medium | | | |
|---|---|---|---|---|
| | 0 µg/ml | <75 µg/ml | 75 µg/ml | 500 µg/ml |
| E. coli K12/pKC203 | + | + | + | N/T |
| Mouse Ltk⁻ | + | + | − | − |
| Mouse Ltk⁻/pKC214 | + | + | + | + |
| Mouse Ltk⁻/pKC215 | + | + | + | − |

Example 7
Construction of plasmids pGD1 and pGD2 and transformants E. coli K12 BE827/pGD1 and E. coli K12 BE827/pGD2

Plasmid pLG669, the construction of which is described in Guarente and Ptashne, 1981, Proc. Nat. Acad. Sci. USA 78(4):2199, contains a unique BamHI restriction site in the cytochrome c gene. Cloning the 7.5 kb Bg/II fragment of plasmid pKC203 into this site, as described below, allows for the expression of the antibiotic hygromycin B resistance gene. The desired insertion is easily carried out since Bg/II fragments contain 5' extensions with the sequence GATC that are identical to 5' extensions of BamHI fragments. Therefore Bg/II fragments and BamHI fragments are compatible and can be ligated directly for the production of recombinants.

Plasmids pGD1 and pGD2 and transformants E. coli K12 BE827/pGD1 and E. coli K12 BE827/pGD2 are constructed in substantial accordance with the teaching of Example 3, except that plasmid pLG669, with the unique BamHI site, is used instead of plasmid pSV5 gpt. Depending upon the orientation of the Bg/II fragment, plasmids of two orientations result. Plasmid pGD1 designates recombinant plasmids in which the Sa/I restriction site is closest to the ura gene. Plasmid pGD2 designates plasmids with the reverse orientation.

Example 8
Construction of Saccharomyces cerevisiae/pGD1

Yeast (Saccharomyces cerevisiae) is typically grown in 1% yeast extract/2% bacto-peptone using conventional microbiological procedures well known in the art. The transformation of plasmid pGD1 into yeast is carried out in substantial accordance with the teaching of Hinnen et al., 1978, Proc. Nat. Acad. Sci. USA 75:1929. Transformants are selected by adding lethal doses of antibiotic hygromycin B to the culture medium.

Example 9
Construction of Saccharomyces cerevisiae/pGD2

The desired transformants are constructed in substantial accordance with the teaching of Example 8, except that plasmid pGD2 is used instead of plasmid pGD1.

Example 10
Construction of plasmids pGD3 and pGD4 and transformants E. coli K12 BE827/pGD3 and E. coli K12 BE827/pGD4

Plasmid pØ4, the construction of which is described in Solnick, 1981, Cell 24:135, contains the major Adenovirus 2 (Ad2) late promoter at map coordinates 15.4 (EcoRI) to 16.6 (HindIII). Cloning the 7.5 kb Bg/II fragment of plasmid pKC203 into the HindIII site of plasmid pØ4 allows for the expression of the antibiotic hygromycin B resistance gene.

The desired construction is conveniently done by adding, in substantial accordance with the teaching of Ullrich et al., 1977, Science 196:1313, HindIII linkers* to the 7.5 kb Bg/II fragment and then ligating the thus modified fragment to HindIII treated plasmid pØ4 by use of T4 DNA ligase. HindIII restriction and T4 DNA ligase enzyme are respectively available with instructions for use from the firms cited in Examples 2 and 3. After the 7.5 kb Bg/II fragment is provided with the HindIII linkers, the ligation of the fragment onto plasmid pØ4 to form plasmids pGD3 and pGD4 is carried out in substantial accordance with the teaching of Example 3. The subsequent transformation into E. coli K12 BE827 to form E. coli K12 BE827/pGD3 and E. coli K12 BE827/pGD4 is also carried out according to the teaching of Example 3.

*HindIII [(d(CCAAGCTTGG)] and other linkers are readily available at:

Collaborative Research Inc.
1365 Main Street
Waltham, MA 02154

As explained in Examples 3 and 7, plasmids of two orientations result depending upon the orientation of the inserted resistance conferring fragment. Plasmid pGD3 designates recombinant plasmids in which the *Sal*I restriction site of the inserted antibiotic hygromycin B conferring fragment is closest to the *Eco*RI site of the Ad2 promoter. Plasmid pGD4 designates plasmids with the reverse orientation.

Example 11
Construction of Mouse Ltk⁻/pGD3
The construction of Mouse Ltk⁻/pGD3 is carried out in substantial accordance with the teaching of Example 4, except that plasmid pGD3 is used instead of plasmid pKC214.

Example 12
Construction of Mouse Ltk⁻/pGD4
The construction of Mouse Ltk⁻/pGD4 is carried out in substantial accordance with the teaching of Example 4, except that plasmid pGD4 is used instead of plasmid pKC215.

Example 13
Construction of plasmid pKC222 and transformant *E. coli* K12 BE827/pKC222
A. Isolation of the 2.75 kb *Sal*I/*Bg*III fragment of plasmid pKC203
About 5 µg of plasmid pKC203 DNA was treated with *Sal*I and *Bg*III restriction enzymes according to the instructions and under the conditions specified by the manufacturer*. A 2.75 kb fragment that contained the genes and control elements for resistance to antibiotics hygromycin B and G418 was recovered by conventional procedures.

*Restriction enzymes and instructions can be obtained from the sources cited in Example 2.

B. Ligation and final construction
About 5 µg of plasmid pKC7, the construction of which is disclosed in Rao and Rogers, 1979, Gene 7:79, were treated with *Sal*I and *Bg*III restriction enzymes. After the enzymes were inactivated by heating at 70°C for 5 minutes, about 1 µg of the DNA was mixed in a 1:1 ratio with the 2.75 kb *Sal*I/*Bg*III fragment of pKC203. The fragments were joined using T4 DNA ligase according to the instructions and under the conditions specified by the manufacture as cited in claim 3. The resulting plasmid pKC222 was transformed into *E. coli* K12, in substantial accordance with the teaching of Example 3, and was shown to confer resistance to antibiotics ampicillin, hygromycin B and G418.

Example 14
Isolation of the hygromycin B resistance conferring 1.51 kb *Sac*I/*Bg*III fragment of plasmid pKC222
The desired DNA fragment was isolated in substantial accordance with the teaching of Example 13A except that *Sac*I, rather than *Sal*I, was used with *Bg*III for the restriction digest.

Example 15
Isolation of the G418 resistance conferring 1.65 kb *Eco*RI/*Sal*I fragment of plasmid pKC222
The desired DNA fragment was isolated in substantial accordance with the teaching of Example 13A except that *Eco*RI, rather than *Bg*III, was used with *Sal*I for the restriction digest.

Example 16
Construction of plasmids pGD10 and pGD11 and transformants *E. coli* K12 BE827/pGD10 and *E. coli* K12 BE827/pGD11
The desired plasmids are constructed in substantial accordance with the teaching of Examples 3 and 10 except that the 1.51 kb *Sac*I/*Bg*III fragment of plasmid pKC222 is used instead of the 7.5 kb *Bg*III fragment of plasmid pKC203 and except that *Bg*III, rather than *Hind*III, linkers are attached to the antibiotic resistance conferring fragment. The 1.51 kb fragment with the *Bg*III linkers is inserted into plasmid pSV5 gpt in substantial accordance with the procedure taught in Example 3.
As explained in Examples 3 and 7, plasmids of two orientations result depending upon the orientation of the inserted antibiotic resistance conferring fragment. Plasmid pGD10 designates recombinant plasmids in which the *Ava*I restriction site of the inserted hygromycin B resistance conferring fragment is closest to the *Hind*III site of the gpt gene. Plasmid pGD11 designates plasmids with the reverse orientation.
Transformation of plasmids pGD10 and pGD11 into *E. coli* K12 BE827 to respectively form *E. coli* K12 BE827/pGD10 and *E. coli* K12 BE827/pGD11 is also carried out in substantial accordance with the teaching of Example 3.

Example 17
Construction of Mouse Ltk⁻/pGD10
The desired construction is carried out in substantial accordance with the teaching of Example 4 except that plasmid pGD10, rather than plasmid pKC214, is used in the transformation procedure. The thus constructed Mouse Ltk⁻/pGD10 can be grown into mass culture.

11

Example 18
Construction of Mouse Ltk⁻/pGD11
The desired construction is carried out in substantial accordance with the teaching of Example 4 except that plasmid pGD11, rather than plasmid pKC214, is used in the transformation procedure. The thus constructed Mouse Ltk⁻/pGD11 can be grown into mass culture.

Example 19
Construction of plasmids pGD12 and pGD13 and transformants *E. coli* K12 BE827/pGD12 and *E. coli* K12 BE827/pGD13
The desired plasmids are constructed in substantial accordance with the teaching of Examples 3 and 10 except that the 1.65 kb *Eco*RI/*Sal*I fragment of plasmid pKC222 is used instead of the 7.5 kb *Bg*III fragment of plasmid pKC203 and except that *Bg*III, rather than *Hind*III, linkers are attached to the antibiotic resistance conferring fragment. The 1.65 kb fragment with *Bg*III linkers is inserted into plasmid pSV5 gpt in substantial accordance with the procedure taught in Example 3.

As explained in Example 16, plasmids of two orientations result. Plasmid pGD12 designates recombinant plasmids in which the *Pst*I restriction site of the inserted antibiotic G418 resistance conferring fragment is closest to the *Hind*III site of the gpt gene. Plasmid pGD13 designates plasmids with the reverse orientation.

Transformation of plasmids pGD12 and pGD13 into *E. coli* K12 BE827 to respectively form *E. coli* K12 BE827/pGD12 and *E. coli* K12 BE827/pGD13 is also carried out in substantial accordance with the teaching of Example 3 except that antibiotic G418, rather than hygromycin B, is used for selection of transformants.

Example 20
Construction of Mouse Ltk⁻/pGD12
The desired construction is carried out in substantial accordance with the teaching of Example 4 except that plasmid pGD12, rather than plasmid pKC214, is used in the transformation procedure and except that antibiotic G418, rather than hygromycin B, is used for selecting transformants. The thus constructed Mouse Ltk⁻/pGD12 can be grown into mass culture.

Example 21
Construction of Mouse Ltk⁻/pGD13
The desired construction is carried out in substantial accordance with the teaching of Example 4 except that plasmid pGD13, rather than plasmid pKC214, is used in the transformation procedure and except that antibiotic G418, rather than hygromycin B, is used for selecting transformants. The thus constructed Mouse Ltk⁻/pGD13 can be grown into mass culture.

Example 22
Construction of plasmids pGD14 and pGD15 and transformants *E. coli* K12 BE827/pGD14 and *E. coli* K12 BE827/pGD15
The 2.75 kb *Sal*I/*Bg*III fragment of plasmid pKC203 was isolated following the procedure described in Example 13. Molecular linkers are then attached according to the teaching of Example 10 except that *Bg*III, rather than *Hind*III, linkers are used. The resulting fragment is then inserted into plasmid pSV5 gpt, in substantial accordance with the procedure taught in Example 3, to form the desired plasmids.

As explained in Example 16, plasmids of two orientations result. Plasmid pGD14 designates recombinant plasmids in which the *Ava*I restriction site of the antibiotic conferring fragment is closest to the *Hind*III site of the gpt gene. Plasmid pGD15 designates plasmids with the reverse orientation.

Transformation of plasmids pGD14 and pGD15 into *E. coli* K12 BE827 to respectively form *E. coli* K12 BE827/pGD14 and *E. coli* K12 BE827/pGD15 is also carried out in substantial accordance with the teaching of Example 3.

Example 23
Construction of Mouse Ltk⁻/pGD14
The desired construction is carried out in substantial accordance with the teaching of Example 4 except that plasmid pGD14, rather than plasmid pKC214, is used in the transformation procedure. The thus constructed Mouse Ltk⁻/pGD14 can be grown into mass culture.

Example 24
Construction of Mouse Ltk⁻/pGD15
The desired construction is carried out in substantial accordance with the teaching of Example 4 except that plasmid pGD15, rather than plasmid pKC214, is used in the transformation procedure. The thus constructed Mouse Ltk⁻/pGD14 can be grown into mass culture.

Example 25
Construction of plasmid pSC701 and transformant *E. coli* K12 BE827/pSC701

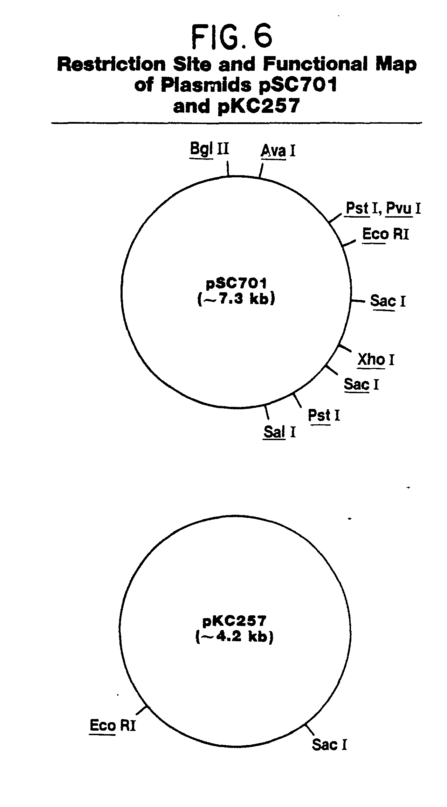

About 5 μl (5 μg) of plasmid pKC203 (isolated in Example 1) in TE buffer (10 mM Tris-HCl, pH 8.0, 1 mM EDTA), 5 μl DTT (100 mM Dithiothreitol), 5 μl (1000 mg/ml) BSA (bovine serum albumin), 25 μl water, 5 μl (5 units) *Bg*/II restriction enzyme, and 5 μl 10× reaction mix* were incubated at 37°C for about 1 hour. The reaction was terminated by incubation at 70°C for 5 minutes and then the reaction mixture was cooled on ice, extracted with each of phenol and chloroform:isoamyl alcohol (24:1), and then ethanol precipitated. The resultant *Bg*/II restriction fragments were dissolved in 5 μl of 5 mM NaCl and then ligated. Ligation was carried out by reacting 1 μl of the *Bg*/II restricted DNA with about 38 μl water, 5 μl (10 mM) ATP, 5 μl ligation mix**, and 1 μl T4 DNA ligase (~$10^5$ New England Bio Lab Units) at 16°C for about 16 hours. The reaction was terminated by incubation at 70°C for 5 minutes. After·cooling on ice, the resultant ligated mixture is used to transform *E. coli* K12 BE827, in substantial accordance with the transformation procedure of Wensink, 1974, on TY plates containing 200 mg/l of antibiotic hygromycin B. Some of the transformants, as conventionally shown by gel electrophoresis (Rao and Rogers, 1978) and other tests, contain only the desired ~7.3 kb plasmid. Such a transformant, designated herein as *E. coli* K12 BE827/pSC701, is selected, plated on TY agar containing 200 μg./ml. of antibiotic hygromycin B, and then cultured using conventional microbiological techniques. The resultant cells are used to isolate plasmid pSC701 according to the procedure of Example 1A. The presence of the antibiotic hygromycin B and G418 resistance genes in plasmid pSC701 was further confirmed by subsequent transformation, selection, and restriction enzyme analysis. A restriction site and functional map of plasmid pSC701 is shown in Figure 6 of the accompanying drawings.

*Reaction mix (10×) for *Bg*/II restriction enzyme was prepared with the following composition:

    600 mM NaCl
    100 mM Tris-HCl, pH 7.4
    100 mM MgCl$_2$

**Ligation mix was prepared with the following composition:

    500 mM Tris-HCl, pH 7.8
    200 mM Dithiothreitol
    100 mM MgCl$_2$

Example 26
Construction of plasmids pKC257 and pKC259 and transformants *E. coli* K12 BE783/pKC257 and *E. coli* K12 BE783/pKC259

The desired plasmid was made in substantial accordance with the teaching of Example 25 except that plasmid pSC701 and *Hae*II restriction enzyme and reaction mix*, rather than plasmid pKC203 and *Bg*/II restriction enzyme and reaction mix, were used. Plasmid pSC701 contains more than one *Hae*II restriction site to therefore *Hae*II digestion and subsequent ligation results in a mixture of different plasmids.

*Reaction mix (10×) for *Hae*II restriction enzyme was prepared with the following composition:

    60 mM Tris-HCl, pH 7.4
    60 mM MgCl$_2$

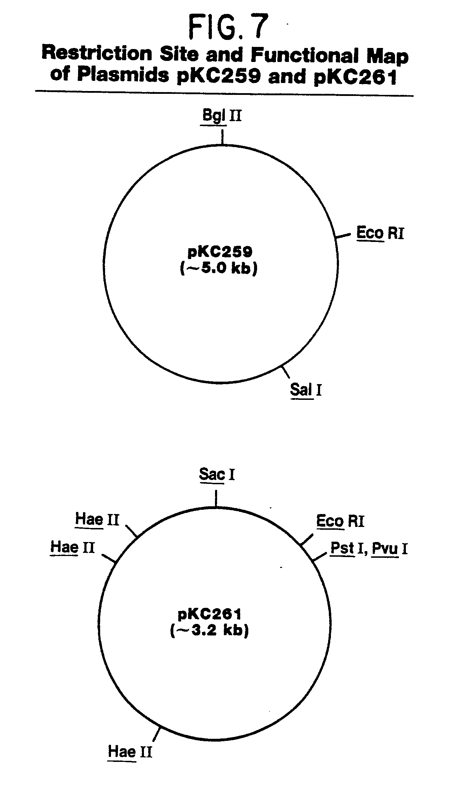

The resultant ligated mixture, which includes the desired hygromycin B resistance-conferring ~4.2 kb plasmid pKC257 and also the hygromycin B, G418, and apramycin resistance-conferring ~5.0 kb plasmid pKC259, was used to transform *E. coli* K12 BE783 (deposited and made part of the permanent stock culture collection of the Northern Regional Research Laboratory, Peoria, Illinois under the accession number B-15020), in substantial accordance with the transformation procedure of Example 25. The desired transformants were selected, plated on TY agar containing 200 μg/ml of antibiotic hygromycin B, and then cultured separately using conventional microbiological techniques. Transformants containing pKC257 were easily and conventionally identified by screening for hygromycin B resistance and transformants containing plasmid pKC259 were identified by screening for apramycin and hygromycin B resistance. The transformants, designated herein as *E. coli* K12 BE783/pKC257 and *E. coli* K12 BE783/pKC259, were respectively used to isolate plasmids pKC257 and pKC259 according to the procedure of Example 1A. The presence of the antibiotic resistance genes in the respective plasmids was further confirmed by subsequent transformation, selection, and restriction enzyme analysis. A restriction site and functional map of each of plasmids pKC257 and pKC259 is shown respectively in Figures 6 and 7 of the accompanying drawings.

Example 27
Construction of plasmid pKC261 and transformant *E. coli* K12 BE783/pKC261

The desired plasmid was made in substantial accordance with the teaching of Example 25 except that plasmid pKC257 and *Sau3* AI restriction enzyme and reaction mix*, rather than plasmid pSC701 and *Bg/*II restriction enzyme and reaction mix, were used. The desired plasmid pKC261 is ~3.2 kb and confers resistance to hygromycin B.

*Reaction mix (10×) for *Sau 3*AI restriction enzyme was prepared with the following composition.

500 mM NaCl
60 mM Tris-HCl, pH 7.5
50 mM MgCl$_2$

The resultant ligated mixture is used to transform *E. coli* K12 BE783 in substantial accordance with the transformation procedure of Example 25. Transformants, designated herein as *E. coli* K12 BE783/pKC261, are selected and used to isolate plasmid pKC261 according to the procedure of Example 1A. The presence of the antibiotic hygromycin B resistance gene in plasmid pKC261 was further confirmed by subsequent transformation, selection, and DNA sequence analysis. A restriction site and functional map of plasmid pKC261 is shown in Figure 7 of the accompanying drawings.

Example 28
Construction of plasmid pKC275 and transformant *E. coli* K12 BE1041/pKC275
A. Partial *Hae*II digestion of plasmid pKC261
About 5 µl (5 µg) of plasmid pKC261 (isolated in Example 27) in TE buffer, 5 µl DTT, 5 µl (1000 mg/ml) BSA, 25 µl water, 5 µl (5 units) *Hae*II restriction enzyme, and 5 µl 10× reaction mix were incubated at 37°C for about 1 hour. After the reaction was terminated by incubation at 70°C for 5 minutes, the reaction mixture was cooled on ice, extracted with each of phenol and chloroform:isoamyl alcohol (24:1), and then ethanol precipitated. The resultant *Hae*II restriction fragments were dissolved in 5 µl of 5 mM NaCl.

B. Isolation of ~394 nt plac fragment containing *Hae*II termini
About 5 µl (5 µg) of plasmid pUR222 (isolated from *E. coli* K12 BE1166/pUR222 in substantial accordance with the teaching of Example 1) in TE buffer was *Hae*II digested in substantial accordance with the teaching of Example 28A except that the reaction mixture was incubated at 37°C for about 2 hours to insure that the digestion was complete and not partial. The resultant *Hae*II restriction fragments were dissolved in 5 µl of 5 mM NaCl.

*E. coli* K12 BE1166/pUR222 is a strain deposited and made part of the permanent stock culture collection, Northern Regional Research Laboratory, Peoria, Illinois. It is available to the public as a preferred source and stock reservoir of plasmid pUR222 under the accession number B-15023.

C. Ligation and transformation
About 4 µl each of plasmid pKC261 and plasmid pUR222 *Hae*II restriction fragments (respectively prepared in Examples 28A and B), 31 µl water, 5 µl (10 mM) ATP, 5 µl ligation mix, and 1 µl T4 DNA ligase (~10$^5$ New England Bio Lab Units) were reacted at 16°C for about 16 hours. After the reaction was terminated by incubation at 70°C for 5 minutes, the resultant ligated mixture was cooled on ice. The DNA was then transformed into *E. coli* K12 BE1041 (deposited and made part of the permanent stock culture collection of the Northern Regional Research Laboratory, Peoria, Illinois under the accession number B-15021), in substantial accordance with the transformation procedure of Example 25. Transformants containing only the desired ~3.6 kb plasmid pKC275 were designated as *E. coli* K12 BE1041/pKC275. Such a transformant was selected, plated, cultured, and used for subsequent plasmid isolations. Both the presence of the ~394 nt plac-containing fragment and the detailed structure of plasmid pKC275 were conventionally determined by gel electrophoretic and restriction enzyme analysis.

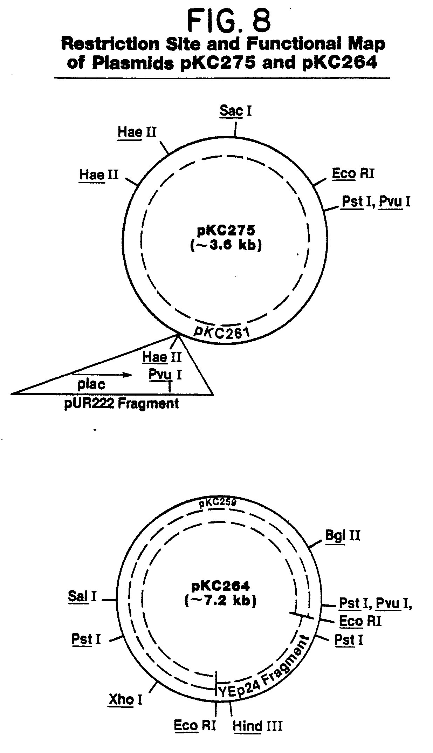

Because plasmid pKC261 has three *Hae*II restriction sites, a partial *Hae*II digestion results in a mixture of different *Hae*II fragments. Consequently, the above illustrative procedure results in the construction of both plasmid pKC275 and also the various insertional isomers of plasmid pKC275. Recombinant plasmids of two orientations are also produced since the ~394 nt plac-containing fragment can be ligated in either direction. Those skilled in the art will understand that the variously oriented plasmid isomers and also the resultant transformants can be readily isolated and identified by conventional means. A restriction site and functional map of plasmid pKC275 is presented in Figure 8 of the accompanying drawings.

Example 29
Construction of plasmid pKC264 and transformant *E. coli* K12 BE783/pKC264
A. *Eco*RI digestion of plasmid pKC259
The desired digestion was carried out in substantial accordance with the teaching of Example 28B except that plasmid pKC259 and *Eco*RI restriction enzyme and reaction mix*, rather than plasmid pUR222 and *Hae*II restriction enzyme and reaction mix, were used. The resultant *Eco*RI restriction fragments were dissolved in 5 µl of 5 mM NaCl.

*Reaction mix (10×) for *Eco*RI restriction enzyme was prepared with the following composition:

    500 mM NaCl
    1000 mM Tris-HCl, pH 7.5
    50 mM $MgCl_2$

B. *Eco*RI digestion of plasmid YEp24 for subsequent isolation of 2 µ DNA

Plasmid YEp24 was isolated from *E. coli* K12 BE1139/YEp24 in substantial accordance with the teaching of Example 1. *E. coli* K12 BE1139/YEp24 is a strain deposited and made part of the permanent stock culture collection of the Northern Regional Research Laboratory, Peoria, Illinois. It is available to the public as a preferred source and stock reservoir of the plasmid under the accession number B-15022. The desired *Eco*RI digestion of plasmid YEp24 was carried out in substantial accordance with the teaching of Example 29A and the resultant *Eco*RI restriction fragments were dissolved in 5 µl of 5 mM NaCl.

C. Ligation and transformation

The *Eco*RI digested plasmid pKC259 and plasmid YEp24 (respectively prepared in Example 29A and B) were ligated and subsequently transformed into *E. coli* K12 BE783 in substantial accordance with the teaching of Example 28C. Transformants containing only the desired ~7.2 kb hygromycin B, apramycin, and G418 resistance-conferring plasmid are designated as *E. coli* K12 BE783/pKC264. Such a transformant is conventionally selected, plated, cultured, and used for subsequent plasmid isolations.

Those skilled in the art will recognize that the 2 µ DNA can be ligated in either direction and that therefore plasmid pKC264 and also plasmids with the reverse orientation result from the above procedure. The various plasmids and resultant transformants can be readily isolated and identified by conventional means. A restriction site and functional map of plasmid pKC264 is presented in Figure 8 of the accompanying drawings.

Example 30
Construction of plasmids pLO314 and pLO315 and transformants *E. coli* K12 BE783/pLO314 and *E. coli* K12 BE783/pLO315
A. *Bgl*II digestion of plasmid pKC259

The desired digestion is carried out in substantial accordance with the teaching of Example 28B except that plasmid pKC259 and *Bgl*II restriction enzyme and reaction mix, rather than plasmid pUR222 and *Hae*II restriction enzyme and reaction mix, is used. The resultant *Bgl*II digest is dissolved in 5 mM NaCl.

B. *Bgl*II digestion of plasmid pSV5 gpt

The desired digestion was carried out in substantial accordance with the teaching of Example 28B except that plasmid pSV5 gpt and *Bgl*II restriction enzyme and reaction mix, rather than plasmid pUR222 and *Hae*II restriction enzyme and reaction mix, was used. The resultant *Bgl*II digest is dissolved in 5 mM NaCl.

C. Ligation and transformation

The desired ligation is carried out in substantial accordance with the teaching of Example 28C except that *Bgl*II digested plasmids pKC259 and pSV5 gpt, rather than plasmids pKC261 and pUR222, are used. The resultant ligated mixture is used to transform *E. coli* K12 BE783 in substantial accordance with the transformation procedure of Wensink, 1974, on TY plates containing 200 µg/ml of antibiotic hygromycin B. Some of the transformants, as can be conventionally shown by gel electrophoresis (Rao and Rogers, 1978) and other tests, contain only the desired plasmid pLO314 or the desired plasmid pLO315. Such transformants are selected, plated, cultured and constitute the desired *E. coli* K12 BE783/pLO314 and *E. coli* K12 BE783/pLO315 transformants. The transformants are used for subsequent isolation of the desired plasmids pLO314 and pLO315.

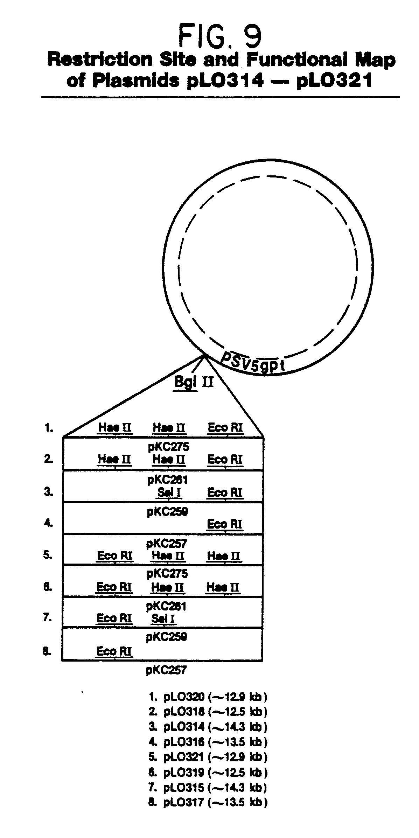

Recombinant plasmids of two orientations result because the DNA fragments are ligated in either direction. Those skilled in the art will understand that the desired plasmids are readily distinguished and identified by the conventional techniques of restriction enzyme and electrophoretic analysis. A restriction site and functional map of each of plasmids pLO314 and pLO315 is presented in Figure 9 of the accompanying drawings.

Example 31
Construction of Mouse Ltk⁻/pLO314

The desired construction is made in substantial accordance with the teaching of Example 4 except that plasmid pLO314, rather than pKC214, is used. The thus constructed Mouse Ltk⁻/pLO314 transformants are then grown into mass culture.

Example 32
Construction of Mouse Ltk⁻/pLO315
    The desired construction is made in substantial accordance with the teaching of Example 4 except that plasmid pLO315, rather than pKC214, is used. The thus constructed Mouse Ltk⁻/pLO315 transformants are then grown into mass culture.

Example 33
Construction of pLO316 and pLO317 and transformants *E. coli* K12 BE783/pLO316 and *E. coli* K12 BE783/pLO317
A. *Sac*I digestion of plasmid pKC257 and addition of *Bg*/II linkers
    The desired digestion is carried out in substantial accordance with the teaching of Example 28B except that plasmid pKC257 and *Sac*I restriction enzyme and reaction mix*, rather than plasmid pUR222 and *Hae*II restriction enzyme and reaction mix, are used.
    The addition of *Bg*/II linkers**, to the *Sac*I digested plasmid pKC257 termini is carried out in substantial accordance with the teaching of St. John *et al.*, 1981, J. Mol. Biol. 152:317.

    *Reaction mix (10×) for *Sac*I restriction enzyme is prepared with the following composition:

        600 mM NaCl
        100 mM Tris-HCl, pH 7.4
        100 mM MgCl₂

    **Bg*/II [(d(CAGATCTG)] and other linkers are readily available at:

        Collaborative Research Inc.
        1365 Main Street
        Waltham, MA 02154

B. Ligation and transformation
    Linear plasmid pKC257 with *Bg*/II termini is ligated to *Bg*/II digested plasmid pSV5 gpt (prepared in Example 30B) in substantial accordance with the teaching of Example 30C. The ligated mixture is used to transform *E. coli* K12 BE783 in the manner also disclosed in Example 30C. The resultant *E. coli* K12 BE783/pLO316 and *E. coli* K12 BE783/pLO317 transformants are used for isolation of the desired plasmids pLO316 and pLO317. As explained in Example 30C, plasmids of two orientations result depending upon the orientation of the inserted resistance-conferring fragment. The plasmids and resultant transformant can be readily distinguished and identified by conventional means. A restriction site and functional map of each of plasmids pLO316 and pLO317 is presented in Figure 9 of the accompanying drawings.

Example 34
Construction of Mouse Ltk⁻/pLO316 and Mouse Ltk⁻/pLO317
    The desired constructions are separately made in substantial accordance with the teaching of Example 4 except that either of plasmids pLO316 or pLO317, rather than plasmid pKC214, is used. The thus constructed Mouse Ltk⁻/pLO316 and Mouse Ltk⁻/pLO317 transformants are then separately grown into mass culture.

Example 35
Construction of plasmids pLO318 and pLO319 and transformants *E. coli* K12 BE783/pLO318 and *E. coli* K12 BE783/pLO319
    The desired constructions are made in substantial accordance with the teaching of Example 33 except that plasmid pKC261, rather than plasmid pKC257, is used. The resultant *E. coli* K12 BE783/pLO318 and *E. coli* K12 BE783/pLO319 transformants are used for isolation of the desired plasmids pLO318 and pLO319. As explained in Example 30C, plasmids of two orientations result depending upon the orientation of the inserted resistance-conferring fragment. The plasmids and resultant transformants can be readily distinguished and identified by conventional means. A restriction site and functional map of each of plasmids pLO318 and pLO319 is presented in Figure 9 of the accompanying drawings.

Example 36
Construction of Mouse Ltk⁻/pLO318 and Mouse Ltk⁻/pLO319
    The desired constructions are separately made in substantial accordance with the teaching of Example 4 except that either of plasmids pLO318 or pLO319, rather than plasmid pKC214, is used. The thus constructed Mouse Ltk⁻/pLO318 and Mouse Ltk⁻/pLO319 transformants are then separately grown into mass culture.

## Example 37
Construction of plasmids pLO320 and pLO321 and transformants *E. coli* K12 BE1041/pLO320 and *E. coli* K12 BE1041/pLO321

The desired constructions are made in substantial accordance with the teaching of Example 33 except that plasmid pKC275, rather than plasmid pKC257, is used. The resultant *E. coli* K12 BE1041/pLO320 and *E. coli* K12 BE1041/pLO321 transformants are used for isolation of the desired plasmids pLO320 and pLO321. As explained in Example 30C, plasmids of two orientations result depending upon the orientation of the inserted resistance-conferring fragment. The plasmids and resultant transformants can be readily distinguished and identified by conventional means. A restriction site and functional map of each of plasmids pLO320 and pLO321 is presented in Figure 9 of the accompanying drawings.

## Example 38
Construction of Mouse Ltk⁻/pLO320 and Mouse Ltk⁻/pLO321

The desired constructions are separately made in substantial accordance with the teaching of Example 4 except that either of plasmids pLO320 or pLO321, rather than plasmid pKC214, is used. The thus constructed Mouse Ltk⁻/pLO320 and Mouse Ltk⁻/pLO321 transformants are then separately grown into mass culture.

## Example 39
Construction of plasmid pKC273 and transformant *E. coli* K12 BE783/pKC273

A. Construction of a 2 μ and ura⁺ gene-containing linear DNA with *Bam*HI and *Sal*I termini

About 5 μl (5 μg) of plasmid pYEp24 in TE buffer, 5 μl DTT, 5 μl (1000 mg/ml) BSA, 25 μl water, 5 μl (5 units) *Bam*HI restriction enzyme, and 5 μl 10× reaction mix* were incubated at 37°C for 1 hour and then at 70°C for 5 minutes. The *Bam*HI digested DNA was cooled on ice, ethanol precipitated, and then dissolved in 30 μl of water to which 5 μl of 10× *Sal*I buffer (reaction mix)* and 5 μl DTT, 5 μl (1000 mg/ml) BSA, and 5 μl (5 units) *Sal*I restriction enzyme were added. The resultant mixture was incubated at 37°C for 1 hour, then at 70°C for 5 minutes, and finally cooled to 4°C. The mixture was then extracted with each of phenol and chloroform:isoamyl alcohol (24:1) and lastly ethanol precipitated. The resultant precipitate contained the desired linear DNA and was dissolved in 5 μl of 5 mM NaCl and stored at 4°C for future use.

*Reaction mix (10×) for *Bam*HI and *Sal*I restriction enzymes were each prepared with the following composition:

1500 mM NaCl
60 mM Tris-HCl, pH 7.9
60 mM MgCl₂

B. Construction of a hygromycin B resistance gene-containing linear DNA with *Bam*HI and *Sal*I termini

The desired linear DNA was constructed in substantial accordance with the teaching of Example 39A except that plasmid pKC259, rather than plasmid YEp24, was used. The resultant precipitate contained the desired linear DNA and was dissolved in 5 μl of 5 mM NaCl and stored at 4°C for future use.

C. Ligation and transformation

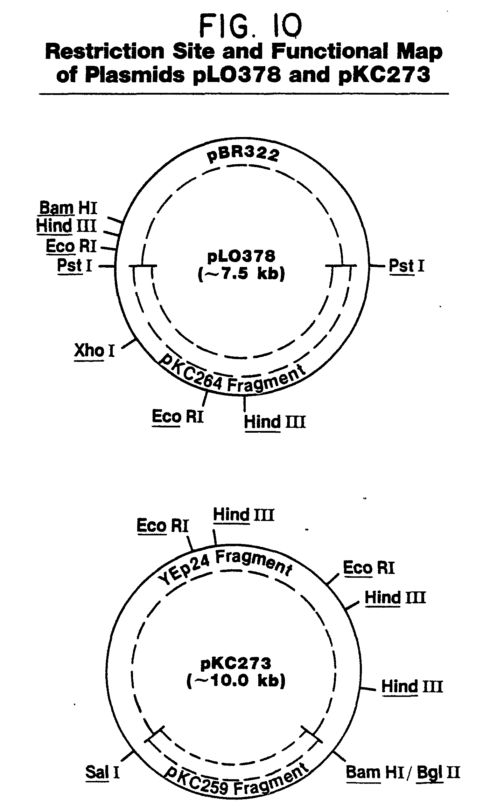

The desired ligation and transformation into *E. coli* K12 BE783 were carried out in substantial accordance with the teaching of Example 28C except that the DNA fragments prepared in Examples 39A and B, rather than the *Hae*II fragments of plasmids pUR222 and pKC261, were ligated and used in subsequent transformations. Some of the resultant transformants, as conventionally shown by selection, gel electrophoresis (Rao and Rogers, 1978) and other tests, contained the desired plasmid. Such a transformant, designated as *E. coli* K12 BE783/pKC273, was selected, plated, cultured, and used for subsequent isolation of plasmid pKC273. A restriction site and functional map of plasmid pKC273 is presented in Figure 10 of the accompanying drawings.

## Example 40
Construction of *Saccharomyces cerevisiae*/pKC273

Yeast (*Saccharomyces cerevisiae*) was grown in 1% yeast extract/2% bacto-peptone/1% glucose using conventional and known microbiological procedures. The transformation of plasmid pKC273 into yeast was carried out in substantial accordance with the teaching of Hinnen *et al.,* 1978, Proc. Nat. Acad. Sci. USA 75:1929. Transformants were selected by their ability to grow on minimal media lacking uracil (Ura⁺ phenotype). Ura⁺ transformants were subsequently tested for their ability to grow on complex media supplemented with 200 μg/ml of hygromycin B. Such a dose is lethal to non-transformed cells. *Saccharomyces cerevisiae*/pKC273 transformant cells grow on these media while untransformed *Saccharomyces cerevisiae* are killed.

Example 41
Construction of plasmid pLO378 and transformant *E. coli* K12 BE783/pLO378
A. *Pst*I digestion of plasmid pBR322
   The desired digestion is carried out in substantial accordance with the teaching of Example 28B except that plasmid pBR322 and *Pst*I restriction enzyme and reaction mix*, rather than plasmid pUR222 and *Hae*II restriction enzyme and reaction mix, is used. The resultant *Pst*I digest is dissolved in 5 µl of 5 mM NaCl.

   *Reaction mix (10×) for *Pst*I restriction enzyme was prepared with the following composition:

   500 mM NaCl
   60 mM Tris-HCl, pH 7.4
   60 mM MgCl$_2$

B. *Pst*I digestion of plasmid pKC264
   The desired digestion was carried out in substantial accordance with the teaching of Example 41A except that plasmid pKC264, rather than plasmid pBR322, was used. The resultant *Pst*I digest was dissolved in 5 µl of 5 mM NaCl.

C. Ligation and transformation
   The desired ligation and transformation into *E. coli* K12 BE783 is carried out in substantial accordance with the teaching of Example 28C except that the DNA fragments prepared in Examples 41A and B, rather than the *Hae*II fragments of plasmids pUR222 and pKC261, are ligated and used in subsequent transformations. Some of the transformants, as can be conventionally shown by antibiotic selection, gel electrophoresis (Rao and Rogers, 1978) and other tests, contain the desired plasmid. Such a transformant, designated as *E. coli* K12 BE783/pLO378, is selected, plated, cultured and used for subsequent isolation of plasmid pLO378.
   As explained in Example 30C, plasmids of two orientations result depending upon the orientation of the inserted resistance-conferring fragment. Therefore, in addition to plasmid pLO378, the above procedure also generates plasmids with the reverse orientation. Those skilled in the art can readily distinguish and identify these plasmids and resultant transformants by conventional means. A restriction site and functional map of plasmid pLO378 is presented in Figure 10 of the accompanying drawings.

Example 42
Construction of *Saccharomyces cerevisiae*/pLO378
   The desired transformation is carried out in substantial accordance with the teaching of Example 40 except that the G418 resistance gene-containing plasmid pLO378, rather than plasmid pKC273, is used. Transformants are identified by conventionally screening the recipient yeast cells for the presence of plasmid DNA. The desired *Saccharomyces cerevisiae*/pLO378 transformants are thus readily identified and isolated.

**Claims**

1. Plasmid pKC222 which contains the ~2.75 kB *Sal*I/*Bgl*II restriction fragment of plasmid pKC203 as obtainable from *E. coli* JR225 ATCC 31912 ligated to the *Sal*I/*Bgl*II restriction fragment of plasmid pKC7, and which confers resistance to antibiotics ampicillin, hygromycin B and G418 when transformed into an *E. coli* cell.

2. A recombinant DNA cloning vector that comprises a eukaryotic promoter, the plasmid pBR322 replicon, and
   a) the ~7.5 kb *Bgl*II restriction fragment of plasmid pKC203 that conveys resistance to antibiotics hygromycin B and G418, or
   b) the ~2.75 kb *Sal*I/*Bgl*II restriction fragment of plasmid pKC203 or plasmid pKC222 that conveys resistance to antibiotics hygromycin B and G418, or
   c) the ~1.51 kb *Bgl*II/*Sac*I restriction fragment of plasmid pKC222 that conveys resistance to the antibiotic hygromycin B, or
   d) the ~1.65 kb *Eco*RI/*Sal*I restriction fragment of plasmid pKC222 that conveys resistance to the antibiotic G418, or
   e) a DNA sequence derived from the said ~7.5 kb restriction fragment that conveys resistance to either or both of antibiotics hygromycin B and G418;
   the resistance to antibiotics hygromycin B and/or G418 being conveyed when the vector is transformed into a host cell that is sensitive to one or both of said antibiotics, the host cell being susceptible to transformation, cell division, and culture; subject to the limitations that the promoter is functional in mouse or yeast cells, that the antibiotic resistance conveying restriction fragments are adjacent to and, in a eukaryotic host cell, transcribed from the promoter, and that the DNA sequences conveying resistance to antibiotic G418 do not code for the enzyme phosphotransferase.

3. The vector of Claim 2 that is a plasmid.

4. The vector of Claim 3 in which the eukaryotic promoter is the early SV40 promoter of plasmid pSV5 gpt.

5. The vector of Claim 4 that is a construct of the ~7.5 kb *Bg*/ll restriction fragment of plasmid pKC203 and the *Bg*/ll digest of plasmid pSV5 gpt which is plasmid pKC214 having the restriction map shown in Figure 3 or its insertional isomer plasmid pKC215 having the restriction map shown in Figure 4.

6. The vector of Claim 4 that is a construct of the ~1.51 kb *Sac*l/*Bg*/ll restriction fragment of plasmid pKC222 provided with a *Bg*/ll linker at the *Sac*l terminus and the *Bg*/ll digest of plasmid pSV5 gpt which is plasmid pGD10 in which the sub-terminal *Ava*l restriction site of the pKC222 fragment is closest to the *Hind*lll restriction site of the gpt gene, or its insertional isomer plasmid pGD11 in which the orientation of the pKC222 fragment is reversed.

7. The vector of Claim 4 that is a construct of the ~1.65 kb *Eco*Rl/*Sa*ll restriction fragment of plasmid pKC222 provided with *Bg*/ll linkers at each terminus and the *Bg*/ll digest of plasmid pSV5 gpt which is plasmid pGD12 in which the sub-terminal *Pst*l restriction site of the pKC222 fragment is closest to the *Hind*lll restriction site of the gpt gene, or its insertional isomer plasmid pGD13 in which the orientation of the pKC222 fragment is reversed.

8. The vector of Claim 4 that is a construct of the ~2.75 kb *Sa*ll/*Bg*/ll restriction fragment of plasmid pKC203 provided with *Bg*/ll linkers at each terminus and the *Bg*/ll digest of plasmid pSV5 gpt which is plasmid pGD14 in which the sub-terminal *Ava*l restriction site of the pKC203 fragment is closest to the *Hind*lll restriction site of the gpt gene, or its insertional isomer plasmid pGD15 in which the orientation of the pKC203 fragment is reversed.

9. The vector of Claim 4 which is plasmid pLO314 having the restriction map shown in Figure 9(3) or its insertional isomer plasmid pLO315 having the restriction map shown in Figure 9(7).

10. The vector of Claim 4 in which is plasmid pLO316 having the restriction map shown in Figure 9(4) or its insertional isomer plasmid pLO317 having the restriction map shown in Figure 9(8).

11. The vector of Claim 4 which is plasmid pLO318 having the restriction map shown in Figure 9(2) or its insertional isomer plasmid pLO319 having the restriction map shown in Figure 9(6).

12. The vector of Claim 4 which is plasmid pLO320 having the restriction map shown in Figure 9(1) or its insertional isomer plasmid pLO321 having the restriction map shown in Figure 9(5).

13. The vector of Claim 13 in which the eukaryotic promoter is the ura+ promoter of plasmid YEp24.

14. The vector of Claim 13 which is plasmid pKC273 having the restriction map shown in Figure 10.

15. The vector of Claim 13 which is plasmid pLO378 having the restriction map shown in Figure 10.

16. An *E. coli* host cell transformed with the recombinant DNA cloning vector of any of Claims 1 to 15.

17. The host cell of Claim 16 which is an *E. coli* K12 or *E. coli* K12 BE783, 827 or 1041 cell.

18. A mouse host cell transformed with the recombinant DNA cloning vector of any of Claims 1 to 12.

19. The host cell of Claim 18 which is a Mouse Ltk⁻ cell.

20. A yeast cell transformed with the recombinant DNA vector of any of Claims 1, 2 or 13 to 15.

21. The host cell of Claim 20 which is a *Saccharomyces cerevisiae* cell.

22. Plasmid pSC701 obtained by self-ligation of the ~7.3 kb *Bg*/ll restriction fragment of plasmid pKC203 (ATCC 31912) and having the restriction map shown in Figure 6.

23. Plasmid pKC257 obtained by incubating plasmid pSC701 with *Hae*ll restriction enzyme, self-ligating the resulting mixture of restriction fragments, transforming an *E. coli* K12 strain with the ligated mixture, and screening the transformants for hygromycin B resistance; and having the molecular weight and restriction map shown in Figure 6.

24. Plasmid pKC259 obtained by incubating plasmid pSC701 with *Hae*ll restriction enzyme, self-ligating the resulting mixture of restriction fragments, transforming an *E. coli* K12 strain with the ligated mixture, and screening the transformants for ampicillin and hygromycin B resistance; and having the molecular weight and restriction map shown in Figure 7.

25. Plasmid pKC261 obtained by self-ligation of the ~3.2 kb *Sau*3Al restriction fragment of plasmid pKC257 and having the restriction map shown in Figure 7.

26. Plasmid pKC275 obtained by ligating the ~396 base plac containing *Hae*ll restriction fragment of plasmid pUR222 as obtainable from *E. coli* K12 BE1166 NRRL B-15023 and a mixture of *Hae*ll restriction fragments of plasmid pKC261, transforming an *E. coli* K12 strain with the ligated mixture, and selecting transformants containing only a ~3.6 kb plasmid; and having the restriction map shown in Figure 8.

27. Plasmid pKC264 obtained by ligating the 2 μ *Eco*Rl restriction fragment of plasmid YEp24 as obtainable *E. coli* K12BE1139 NRRL B-15022 and a mixture of *Eco*Rl restriction fragments of plasmid pKC259, transforming an *E. coli*, K12 strain with the ligated mixture, and selecting transformants containing an ~7.2 kb hygromycin B, apramycin, and G418 resistance-conferring plasmid; and having the restriction map shown in Figure 8.

28. The ~7.5 kb *Bg*/ll restriction fragment of plasmid pKC203.

29. The ~2.75 kb *Sa*ll/*Bg*/ll restriction fragment of plasmid pKC203.

30. The ~1.51 kb *Sac*l/*Bg*/ll restriction fragment of plasmid pKC222.

31. The ~1.65 kb *Eco*Rl/*Sa*ll restriction fragment of plasmid pKC222.

# EP 0 068 740 B1

**Patentansprüche**

1. Plasmid pKC222, das das ~2,75 kb-SalI/BglII-Restriktionsfragment des Plasmids pKC203, das aus Escherichia coli JR225 ATCC 31912 erhältlich ist, welches an das SalI/BglII-Restriktionsfragment des Plasmids pKC7 gebunden enthält, und das eine Resistenz gegen die Antibiotika Ampicillin, Hygromycin B und G418 verleiht, wenn es in eine Zelle von Escherichia coli transformiert ist.

2. Rekombinanter DNA-Klonierungsvektor, umfassend einen eukaryotischen Promoter, des Replikon des Plasmids pBR322 und

a) das ~7,5 kb-BglII-Restriktionsfragment des Plasmids pKC203, das Resistenz gegen die Antibiotika Hygromycin B und G418 verleiht, oder

b) das ~2,75 kb-SalI/BglII-Restriktionsfragment des Plasmids pKC203 oder des Plasmids pKC222, das Resistenz gegen die Antibiotika Hygromycin B und G418 verleiht, oder

c) das ~1,51 kb-BglII/SacI-Restriktionsfragment des Plasmids pKC222, das Resistenz gegen das Antibiotikum Hygromycin B verleiht, oder

d) das ~1,65 kb-EcoRI/SalI-Restriktionsfragment des Plasmids pKC222, das Resistenz gegen das Antibiotikum G418 verleiht, oder

e) eine DNA Sequenz, die vom obigen ~7,5 kb-Restriktionsfragment abgeleitet ist, welche Resistenz gegen eines oder beide der Antibiotika Hygromycin B und G418 verleiht,

wobei die Resistenz gegen die Antibiotika Hygromycin B und/oder G418 verliehen wird, wenn der Vektor in eine Wirtszelle transformiert ist, die auf eines oder beide dieser Antibiotika empfindlich ist, die Wirtszelle einer Transformation, Zellteilung und Züchtung zugänglich ist, mit den Maßgaben, daß der Promotor in Mauszellen oder Hefezellen funktional ist, daß die Antibiotikaresistenz verleihenden Restriktionsfragmente benachbart zum und in einer eukaryotischen Wirtszelle transkribiert vom Promotor angeordnet sind und daß die DNA Sequenzen, welche Resistenz gegen das Antibiotikum G418 verleihen, nicht für das Enzym Phosphotransferase codieren.

3. Vektor nach Anspruch 2, dadurch gekennzeichnet, daß er ein Plasmid ist.

4. Vektor nach Anspruch 3, worin der eukaryotische Promotor der frühe SV40-Promotor des Plasmids pSV5 gpt ist.

5. Vektor nach Anspruch 4, dadurch gekennzeichnet, daß er ein Konstrukt aus dem ~7,5 kb-BglII-Restriktionsfragment des Plasmids pKC203 und dem BglII-Abbauprodukt des Plasmids pSV5 gpt ist, bei dem es sich um das Plasmid pKC214 mit dem in der Figur 3 gezeigten Restriktionsplan oder um dessen insertionsisomeres Plasmid pKC215 mit dem in Figur 4 gezeigten Restriktionsplan handelt.

6. Vektor nach Anspruch 4, dadurch gekennzeichnet, daß er ein Konstrukt aus dem ~1,51 kb-SacI/BglII-Restriktionsfragment des Plasmids pKC222, das am SacI-Terminus einen BglII-Linker aufweist, und aus dem BglII-Abbauprodukt des Plasmids pSV5 gpt ist, bei dem es sich um das Plasmid pGD10 handelt, bei welchem sich die subterminale AvaI-Restriktionsstelle des pKC222 Fragments sehr nahe zur HindIII-Restriktionsstelle des gpt-Gens befindet, oder um dessen insertionsisomers Plasmid pGD11 handelt, bei welchem die Orientierung des pKC222-Fragments umgekehrt ist.

7. Vektor nach Anspruch 4, dadurch gekennzeichnet, daß er ein Konstrukt aus dem ~1,65 kb-EcoRI/SalI-Restriktionsfragment des Plasmids pKC222, das an jedem Terminus BglII-Linker aufweist, und aus dem BglII-Abbauprodukt des Plasmids pSV5 gpt ist, bei dem es sich um das Plasmid pGD12 handelt, bei welchem sich die subterminale PstI-Restriktionsstelle des pKC222-Fragments sehr nahe zur HindIII-Restriktionsstelle des gpt-Gens befindet, oder um dessen Insertionsisomers Plasmid pGD13 handelt, bei welchem die Orientierung des pKC222 Fragments umgekehrt ist.

8. Vektor nach Anspruch 4, dadurch gekennzeichnet, daß er ein Konstrukt aus dem ~2,75 kb-SalI/BglII-Restriktionsfragment des Plasmids pKC203, das an jedem Terminus BglII-Linker aufweist, und aus dem BglII-Abbauprodukt des Plasmids pSV5 gpt ist, bei dem es sich um das Plasmid pGD14 handelt, bei welchem sich die subterminale AvaI-Restriktionsstelle des pKC203-Fragments sehr nahe zur HindIII-Restriktionsstelle des gpt-Gens befindet, oder um dessen Insertionsisomers Plasmid pGD15 handelt, bei welchem die Orientierung des pKC203 Fragments umgekehrt ist.

9. Vektor nach Anspruch 4, dadurch gekennzeichnet, daß es sich hierbei um das Plasmid pLO314 mit dem in der Figur 9(3) gezeigten Restriktionsplan oder um dessen Insertionsisomeres Plasmid pLO315 mit dem in der Figur 9(7) gezeigten Restriktionsplan handelt.

10. Vektor nach Anspruch 4, dadurch gekennzeichnet, daß es sich hierbei um das Plasmid pLO316 mit dem in der Figur 9(4) gezeigten Restriktionsplan oder um dessen Insertionsisomeres Plasmid pLO317 mit dem in der Figur 9(8) gezeigten Restriktionsplan handelt.

11. Vektor nach Anspruch 4, dadurch gekennzeichnet, daß es sich hierbei um das Plasmid pLO318 mit dem in der Figur 9(2) gezeigten Restriktionsplan oder um dessen Insertionsisomeres Plasmid pLO319 mit dem in der Figur 9(6) gezeigten Restriktionsplan handelt.

12. Vektor nach Anspruch 4, dadurch gekennzeichnet, daß es sich hierbei um das Plasmid pLO320 mit dem in der Figur 9(1) gezeigten Restriktionsplan oder um dessen Insertionsisomeres Plasmid pLO321 mit dem in der Figur 9(5) gezeigten Restriktionsplan handelt.

13. Vektor nach Anspruch 3, worin der eukaryotische Promotor der ura+-Promotor des Plasmids YEp24 ist.

20

# EP 0 068 740 B1

14. Vektor nach Anspruch 13, dadurch gekennzeichnet, daß es sich hierbei um das Plasmid pKC273 mit dem in der Figur 10 gezeigten Restriktionsplan handelt.

15. Vektor nach Anspruch 13, dadurch gekennzeichnet, daß es sich hierbei um das Plasmid pLO378 mit dem in der Figur 10 gezeigten Restriktionsplan handelt.

16. Escherichia coli-Wirtszelle, transformiert mit dem reckombinanten DNA-Klonierungsvektor nach einem der Ansprüche 1 bis 15.

17. Wirtszelle nach Anspruch 16, dadurch gekennzeichnet, daß es sich hierbei um eine Zelle von Escherichia coli K12 oder Escherichia coli K12 BE783, 827 oder 1041 handelt.

18. Maus-Wirtszelle, transformiert mit dem rekombinanten DNA-Klonierungsvektor nach einem der Ansprüche 1 bis 12.

19. Wirtszelle nach Anspruch 18, dadurch gekennzeichnet, daß es sich hierbei um eine Ltk⁻ Zelle der Maus handelt.

20. Hefezelle, transformiert mit dem reckombinanten DNA-Vektor nach einem der Ansprüche 1, 2 oder 13 bis 15.

21. Wirtszelle nach Anspruch 20, dadurch gekennzeichnet, daß es sich hierbei um eine Zelle von Saccharomyces cerevisiae handelt.

22. Plasmid pSC701, erhalten durch Selbstverknüpfung des ~7,3 kb-BglIII-Restriktionsfragments des Plasmids pKC203 (ATCC 31912) und mit dem in der Figur 6 gezeigten Restriktionsplan.

23. Plasmid pKC257, erhalten durch Inkubation des Plasmids pSC701 mit HaeII-Restriktionsenzym, Selbstverknüpfung des erhaltenen Gemisches der Restriktionsfragmente, Transformierung eines Stammes von Escherichia coli K12 mit dem verknüpften Gemisch und Auslese der Transformanten mit Resistenz gegen Hygromycin B, und mit dem in der Figur 6 gezeigten Restriktionsplan und dem darin angegebenen Molekulargewicht.

24. Plasmid pKC259, erhalten durch Inkubation des Plasmids pSC701 mit HaeII-Restriktionsenzym, Selbstverknüpfung des erhaltenen Gemisches der Restriktionsfragmente, Transformierung eines Stammes von Escherichia coli K12 mit dem verknüpften Gemisch und Auslese der Transformanten mit Resistenz gegen Ampicillin und Hygromycin B, und mit dem in der Figur 7 gezeigten Restriktionsplan und dem darin angegebenen Molekulargewicht.

25. Plasmid pKC261, erhalten durch Selbstverknüpfung des ~3,2 kb-Sau3AI-Restriktionsfragment des Plasmids pKC257, und mit dem in der Figur 7 gezeigten Restriktionsplan.

26. Plasmid pKC275, erhalten durch Verknüpfung des plac enthaltenden ~396 kb-HaeII-Restriktionsfragments des Plasmids pUR222, welches aus Escherichia coli K12 BE1166 (NRRL B-15 023) erhältlich ist, mit einem Gemisch von HaeII-Restriktionsfragmenten des Plasmids pKC261, Transformierung eines Stammes von Escherichia coli K12 mit dem verknüpften Gemisch und Selektierung der Transformanten, die lediglich ein ~3,6 kb-Plasmid enthalten, und mit dem in der Figur 8 gezeigten Restriktionsplan.

27. Plasmid pKC264, erhalten durch Verknüpfung des 2μ-EcoRI-Restriktionsfragments des Plasmids YEp24, das aus Escherichia coli K12 BE1139 (NRRL B-15022) erhältlich ist, und eines Gemisches von EcoRI-Restriktionsfragmenten des Plasmids pKC259, Transformieren eines Stammes von Escherichia coli K12 mit dem verknüpften Gemisch und Selektieren der Transformanten, die ein ~7,2 kb-Plasmid enthalten, des Resistenz gegen Hygromycin B, Apramycin und G418 verleiht, und mit dem in der Figur 8 gezeigten Restriktionsplan.

28. ~7,5 kb-BglIII-Restriktionsfragment des Plasmids pKC203.

29. ~2,5 kb-SalI/BglIII-Restriktionsfragment des Plasmids pKC203.

30. ~1,51 kb-SacI/BglIII-Restriktionsfragment des Plasmids pKC222.

31. ~1,65 kb-EcoRI/SalI-Restriktionsfragment des Plasmids pKC222.

## Revendications

1. Plasmide pKC222 contenant le fragment de restriction ~2,75 kb *SalI/Bgl*II du plasmide pKC203 tel que celui pouvant être obtenu à partir d'*E. coli* JR225 ATCC 31912 ligaturé au fragment de restriction *SalI/Bgl*II du plasmide pKC7 et conférant une résistance aux antibiotiques ampicilline, hygromycine B et G418 lors de sa transformation en une cellule d'*E. coli*.

2. Vecteur de clonage d'acide désoxyribonucléique (ADN) recombinant comprenant un promoteur eucaryotique, le réplicon du plasmide pBR322, et

a) le fragment de restriction ~7,5 kb *Bgl*II du plasmide pKC203 transmettant sa résistance aux antibiotiques hygromycine B et G418, ou

b) le fragment de restriction ~2,75 kb *SalI/Bgl*II du plasmide pKC203 ou du plasmide pKC222 transmettant sa résistance aux antibiotiques hygromycine B et G418, ou

c) le fragment de restriction ~1,51 kb *Bgl*II/*Sac*I du plasmide pKC222 transmettant sa résistance à l'antibiotique hygromycine B, ou

d) le fragment de restriction ~1,65 kb *Eco*RI/*Sal*I du plasmide pKC222 transmettant sa résistance à l'antibiotique G418, ou

e) une séquence d'ADN dérivant de ce fragment de restriction ~7,5 kb transmettant sa résistance à l'un ou l'autre ou aux deux antibiotiques hygromycine B et G418;

21

la résistance aux antibiotiques hygromycine B et/ou G418 étant transmise lorsque le vecteur est transformé en une cellule hôte qui est sensible à un ou aux deux antibiotiques précités, la cellule hôte étant susceptible à la transformation, à la division des cellules et à la culture; tout en faisant l'objet des limitations selon lesquelles le promoteur est fonctionnel dans des cellules de souris ou de levure, que les fragments de restriction transmettant une résistance aux antibiotiques sont adjacents à et, dans une cellule hôte eucaryotique, transcrits à partir du promoteur, et que les séquences d'ADN conférant une résistance à l'antibiotique G418 n'encodent pas l'enzyme phosphotransférase.

3. Vecteur selon la revendication 2, caractérisé en ce qu'il est un plasmide.

4. Vecteur selon la revendication 3, caractérisé en ce que le promoteur eucaryotique est le promoteur précoce SV40 du plasmide pSV5 gpt.

5. Vecteur selon la revendication 4, caractérisé en ce qu'il est un produit de construction du fragment de restriction ~7,5 kb *Bgl*II du plasmide pKC203 et du produit de digestion *Bgl*II du plasmide pSV5 gpt, lequel est le plasmide pKC214 ayant la carte de restriction illustrée en figure 3 ou son plasmide isomère insertionnel pKC215 ayant la carte de restriction illustrée en figure 4.

6. Vecteur selon la revendication 4, caractérisé en ce qu'il est un produit de construction du fragment de restriction ~1,51 kb *Sacl/Bgl*II du plasmide pKC222 fourni avec une liaison *Bgl*II à la terminaison *Sacl* et le produit de digestion *Bgl*II du plasmide pSV5 gpt qui est le plasmide pGD10 dans lequel le site de restriction *Aval* sub-terminal du fragment pKC222 est le plus proche du site de restriction *Hind*III du gène gpt, ou son plasmide isomère insertionnel pGD11 dans lequel l'orientation du fragment pKC222 est inversée.

7. Vecteur selon la revendication 4, caractérisé en ce qu'il est un produit de construction du fragment de restriction ~1,65 kb *Eco*RI/*Sal*I du plasmide pKC222 fourni avec les liaisons *Bgl*II à chaque terminaison et le produit de digestion *Bgl*II du plasmide pSV5 gpt qui est le plasmide pGD12 dans lequel le site de restriction sub-terminale *Pst*I du fragment pKC222 est le plus proche du site de restriction *Hind*III du gène gpt, on son plasmide isomère insertionnel pGD13 dans lequel l'orientation du fragment pKC222 est inversée.

8. Vecteur selon la revendication 4, caractérisé en ce qu'il est un produit de construction du fragment de restriction ~2,75 kb *Sal*I/*Bgl*II du plasmide pKC203 pourvu des liaisons *Bgl*II à chaque terminaison et du produit de digestion *Bgl*II du plasmide pSV5 gpt qui est le plasmide pGD14 dans lequel le site de restriction sub-terminal *Aval* du fragment pKC203 est le plus proche du site de restriction *Hind*III du gène gpt, ou son plasmide isomère insertionnel pGD15 dans lequel l'orientation du fragment pKC203 est inversée.

9. Vecteur selon la revendication 4, caractérisé en ce qu'il est le plasmide pLO314 ayant la carte de restriction illustrée en figure 9(3) ou son plasmide isomère insertionnel pLO315 ayant la carte de restriction illustrée en figure 9(7).

10. Vecteur selon la revendication 4, caractérisé en ce qu'il est le plasmide pLO316 ayant la carte de restriction représentée en figure 9(4) ou son plasmide isomère insertionnel pLO317 ayant la carte de restriction illustrée en figure 9(8).

11. Vecteur selon la revendication 4, caractérisé en ce qu'il est le plasmide pLO318 ayant la carte de restriction illustrée en figure 9(2) ou son plasmide isomère insertionnel pLO319 ayant la carte de restriction illustrée en figure 9(6).

12. Vecteur selon la revendication 4, caractérisé en ce qu'il est le plasmide pLO320 ayant la carte de restriction illustrée en figure 9(1) ou son plasmide isomère insertionnel pLO321 ayant la carte de restriction illustrée en figure 9(5).

13. Vecteur selon la revendication 3, caractérisé en ce que le promoteur eucaryotique est le promoteur ura+ du plasmide YEp24.

14. Vecteur selon la revendication 13, caractérisé en ce qu'il est le plasmide pKC273 ayant la carte de restriction illustrée en figure 10.

15. Vecteur selon la revendication 13, caractérisé en ce qu'il est le plasmide pLO378 ayant la carte de restriction illustrée en figure 10.

16. Cellule hôte d'*E. coli* transformée avec le vecteur de clonage d'ADN recombinant selon l'une quelconque des revendications 1 à 15.

17. Cellule hôte selon la revendication 16, à savoir une cellule d'*E. coli* K12 ou d'*E. coli* K12 BE783, 827 ou 1041.

18. Cellule hôte de souris transformée avec le vecteur de clonage d'ADN recombinant selon l'une quelconque des revendications 1 à 12.

19. Cellule hôte selon la revendication 18, caractérisée en ce qu'elle est la cellule hôte de souris Ltk⁻.

20. Cellule de levure transformée avec le vecteur d'ADN recombinant selon l'une quelconque des revendications 1, 2 ou 13 à 15.

21. Cellule hôte selon la revendication 20, caractérisée en ce qu'elle est une cellule de *Saccharomyces cerevisiae*.

22. Plasmide pSC701 obtenu par auto-ligature du fragment de restriction ~7,3 kb *Bgl*II du plasmide pKC203 (ATCC 31912) et ayant la carte de restriction illustrée en figure 6.

23. Plasmide pKC257 obtenu par incubation du plasmide pSC701 avec l'enzyme de restriction *Hae*II, auto-ligature du mélange obtenu des fragments de restriction, transformation d'une souche d'*E. coli* K12 avec le mélange ligaturé et sélection des transformants pour la résistance à l'hygromycine B; tout en ayant le poids moléculaire et la carte de restriction comme illustré en figure 6.

24. Plasmide pKC259 obtenu par incubation du plasmide pSC701 avec l'enzyme de restriction *Hae*II, auto-ligature du mélange obtenu des fragments de restriction, transformation d'une souche d'*E. coli* K12 avec le mélange ligaturé et sélection des transformants pour la résistance à l'ampicilline et à l'hygromycine B; tout en ayant le poids moléculaire et la carte de restriction comme illustré en figure 7.

25. Plasmide pKC261 obtenu par auto-ligature du fragment de restriction ~3,2 kb *Sau*3AI du plasmide pKC257 et ayant la carte de restriction illustrée en figure 7.

26. Plasmide pKC275 obtenu par ligature du fragment de restriction *Hae*II contenant ~396 plac base du plasmide pUR222 tel que celui pouvant être obtenu à partir d'*E. coli* K12 BE1166 NRRL B-15023 et d'un mélange de fragments de restriction *Hae*II du plasmide pKC261, en transformant une souche d'*E. coli* K12 avec le mélange ligaturé et en sélectionnant les transformants ne contenant qu'un plasmide ~3,6 kb; et ayant la carte de restriction illustrée en figure 8.

27. Plasmide pKC264 obtenu en ligaturant le fragment de restriction *Eco*Ri 2 μ du plasmide YEp24 tel que celui pouvant être obtenu à partir d'*E. coli* K12 BE1139 NRRL B-15022 et un mélange de fragments de restriction *Eco*RI du plasmide pKC259, en transformant une souche *E. coli* K12 avec le mélange ligaturé et en sélectionnant les transformants contenant un plasmide conférant une résistance à G418, à l'apramycine et à l'hygromycine B ~7,2 kb, tout en ayant la carte de restriction illustrée en figure 8.

28. Fragment de restriction de *Bg*lI ~7,5 kb du plasmide pKC203.

29. Fragment de restriction de *Sa*lI/*Bg*lI ~2,75 kb du plasmide pKC203.

30. Fragment de restriction de *Sac*I/*Bg*lI ~1,51 kb du plasmide pKC222.

31. Fragment de restriction de *Eco*Ri/*Sa*lI ~1,65 kb du plasmide pKC222.

# FIG. I

# Restriction Site and Functional Map of Plasmid pKC203

# FIG. 2·
## Restriction Site and Functional Map of Plasmid pSV5 gpt

PY Tag —Polyoma Sequence
—SV40 Sequence

# FIG. 3
# Restriction Site and Functional
# Map of Plasmid pKC214

PY Tag —Polyoma Sequence
—SV40 Sequence

# FIG. 4
# Restriction Site and Functional Map of Plasmid pKC215

PY Tag   —Polyoma Sequence
         —SV40 Sequence

# FIG. 5
# Restriction Site and Functional Map of Plasmid pKC222

# FIG. 6

## Restriction Site and Functional Map of Plasmids pSC701 and pKC257

# FIG. 7

## Restriction Site and Functional Map of Plasmids pKC259 and pKC261

# FIG. 8
## Restriction Site and Functional Map
## of Plasmids pKC275 and pKC264

# FIG. 9
## Restriction Site and Functional Map
## of Plasmids pLO314 — pLO321

PSV5gpt

Bgl II

| | Hae II | Hae II | Eco RI |
|---|---|---|---|
| 1. | | pKC275 | |
| 2. | Hae II | Hae II | Eco RI |
| | | pKC261 | |
| 3. | | Sal I | Eco RI |
| | | pKC259 | |
| 4. | | | Eco RI |
| | | pKC257 | |
| 5. | Eco RI | Hae II | Hae II |
| | | pKC275 | |
| 6. | Eco RI | Hae II | Hae II |
| | | pKC261 | |
| 7. | Eco RI | Sal I | |
| | | pKC259 | |
| 8. | Eco RI | | |

pKC257

1. pLO320 (~12.9 kb)
2. pLO318 (~12.5 kb)
3. pLO314 (~14.3 kb)
4. pLO316 (~13.5 kb)
5. pLO321 (~12.9 kb)
6. pLO319 (~12.5 kb)
7. pLO315 (~14.3 kb)
8. pLO317 (~13.5 kb)

9

# FIG. 10
## Restriction Site and Functional Map
## of Plasmids pLO378 and pKC273